# EUROPEAN PATENT APPLICATION

(11) **EP 4 659 753 A1**
(43) Date of publication of application: **10.12.2025**
(21) Application number: 24305899.7
(22) Date of filing: 07.06.2024
(51) Int. Cl.: A61K 31/711, A61K 35/74, A01N 63/20, A61K 38/16, A61P 31/00, A61P 31/04, C12N 1/20, A61K 48/00, A61K 35/76

(54) **THERAPEUTIC AND NON-THERAPEUTIC USES OF TAILOCINS OF PSEUDOMONAS SPECIES AGAINST E. COLI AND NEW TAILOCINS OF PSEUDOMONAS FLUORESCENS**

(71) Applicant: Greenphage, 34830 Clapiers (FR)
(72) Inventor: COSTECHAREYRE, Denis, 34130 MAUGUIO (FR); GRAINDORGE, Marie, 34270 LES MATELLES (FR)
(74) Representative: Regimbeau

(57) **Abstract**

The present invention relates to antibacterial compounds efficient against *Escherichia coli,* i.e. a composition comprising a cell lysate, wherein the cell produces at least one tailocin from a *Pseudomonas* bacterium selected from the group consisting of *Pseudomonas fluorescens, Pseudomonas chlororaphis, Pseudomonas koreensis* and *Pseudomonas syringae* species, for use in the prevention or the treatment in a subject of a contamination or an infection by an *Escherichia coli* strain. It further relates to non-therapeutic uses of the same cell lysate for the prevention and/or the treatment of a contamination by an *Escherichia coli* strain in a food product or on a surface, and intimate care product or a food supplement comprising the cell lysate, as well as new nucleic acids encoding one or two original tailocins, expression vectors or cells comprising them, a cell lysate and its therapeutic and non-therapeutic uses.

## Description

### TECHNICAL FIELD OF THE INVENTION

The present invention relates to antibacterial compounds efficient against *Escherichia coli,* i.e. a composition comprising a cell lysate, wherein the cell produces at least one tailocin from a *Pseudomonas* bacterium selected from the group consisting of *Pseudomonas fluorescens, Pseudomonas chlororaphis, Pseudomonas koreensis* and *Pseudomonas syringae* species, for use in the prevention or the treatment in a subject of a contamination or an infection by an *Escherichia coli* strain. It further relates to non-therapeutic uses of the same cell lysate for the prevention and/or the treatment of a contamination by an *Escherichia coli* strain in a food product or on a surface, and intimate care product or a food supplement comprising the cell lysate, as well as new nucleic acids encoding one or two original tailocins, expression vectors or cells comprising them, a cell lysate and its therapeutic and non-therapeutic uses.

### BACKGROUND ART

*Escherichia coli* (*E. coli*) is a Gram-negative, rod-shaped bacterium that is commonly found in the lower intestine of warm-blooded organisms. Most *E. coli* strains are harmless, but some pathogenic variants can cause serious intestinal or extraintestinal diseases largely from their expression of multiple specialized virulence factors (Kaper et al., 2004). Three general clinical syndromes can result from *E. coli* infection: enteric/diarrhoeal disease, urinary tract infections (UTIs) and sepsis/meningitis.

Urinary tract infection (UTI) is the most common extraintestinal *E. coli* infection and the most prevalent bacterial infection in women and elderly individuals (Fazly Bazzaz et al., 2021). Uropathogenic *E. coli* (UPEC) strains are responsible for approximately 90% of UTI seen in individuals with ordinary anatomy, with 20 - 40% experiencing recurrence within 6 - 12 months. In addition, catheter-associated urinary tract infections constitute the majority of nosocomial urinary tract infections (Loubet et al., 2020). This poses significant clinical challenges, particularly with regards to the continuing increase in antibiotic resistance displayed by UPEC. Indeed, resistance to trimethoprim-sulfamethoxazole, widely used as the first-line antimicrobial in the treatment of UTI, as well as fluoroquinolones, used in the treatment of more severe infections, is observed in many countries (Kot, 2019). To date, clone ST131 is the most globally dominant high-risk UPEC clone. This epidemic clone is associated with resistance to multiple antibiotic classes, including fluoroquinolones, third-generation cephalosporins and more recently last-line carbapenems and polymyxins (Nicolas-Chanoine et al., 2008). Altogether, UTI are associated with substantial healthcare and societal costs (7 million clinic visits annually, with a cost exceeding $1.6 billion, in the USA)(Sheerin, 2011). Furthermore, *E. coli* is frequently associated with increasing foodborne illness outbreaks. Intestinal pathogens are divided into six well-described categories: i) enteropathogenic *E. coli* (EPEC), ii) enterohaemorrhagic *E. coli* (EHEC), iii) enterotoxigenic *E. coli* (ETEC), iv) enteroaggregative *E. coli* (EAEC), v) enteroinvasive *E. coli* (EIEC) and vi) diffusely adherent *E. coli* (DAEC). Among the several strains of *E. coli* involved in foodborne illness outbreaks, O157:H7 is the most frequently isolated serovar because of the seriousness of the prognosis of the disease it causes (Alharbi et al., 2022). Cases with non-O157 serogroups such as O26, O111, 0103, 0121, O45, and 0145 have also been increasingly associated with foodborne illness, accounting for about 83% of all shiga toxigenic *E. coli* (STEC) infections in humans from 2000 to 2010 in the United States (Bumunang et al., 2023). Despite legislative and microbiological prevention strategies to ensure food safety and public health, *E*. *coli*-associated diseases remain a global public health challenge worldwide.

*E. coli* is intrinsically susceptible to almost all clinically relevant antimicrobial agents, but this bacterial species has a great capacity to accumulate resistance genes, mostly through horizontal gene transfer. Since the early 2000s, multidrug resistance in *E. coli* has become a worrying issue that is increasingly observed in human but also in veterinary medicine worldwide. In addition to difficult-to-treat infections, resistant *E. coli* constitutes major and shared reservoir of resistance determinants to most families of antimicrobial agents (Poirel et al., 2018). Thus, in the light of the worldwide antimicrobial resistance crisis and under the "One Health" framework, new substitutes to antibiotics are urgently needed.

Tailocins - also called phage tail-like bacteriocin particles - have obtained increasing interest in the last decade for their potential use as antibacterial agent. Tailocins are high-molecular-weight bacteriocins, encoded by bacterial genomes, showing structural similarities with bacteriophage tails. They consist of a contractile (R-type) or non-contractile (F-type) sheath forming a rigid tube attached to a baseplate with a spike-shaped protein complex at its tip and associated receptor binding protein (RBP). These nanomachines kill upon contact with their targeted bacteria by puncturing their membranes leading to cell lysis (Ghequire and Mot, 2015). Their specificity is conferred by cell surface receptors of the target bacterium, notably the lipopolysaccharide (LPS), and receptor-binding proteins on tailocin tail fibers. As with phages, killing activity of tailocins is largely described as directed against closely related strains with an intra-species bactericidal action specific to a subset of strains. In *E. coli,* the production of such tailocins has never been experimentally described, and no intra- or inter-species bactericidal activity via the synthesis of high-molecular-weight bacteriocins has ever been recorded.

### SUMMARY OF THE INVENTION

In the context of the present invention, the inventors surprisingly found that a panel of tailocins produced by various *Pseudomonas* strains of the *Pseudomonas fluorescens, Pseudomonas chlororaphis, Pseudomonas koreensis* and *Pseudomonas syringae* species can unexpectedly show inter-order bactericidal activity on *E. coli,* in addition to intra-genus bactericidal activity on other *Pseudomonas* bacteria, in particular *P. aeruginosa.* In addition, the genome of the bacterial producer showing one of the widest and strongest activities against *E. coli* was sequenced and shown to contain an original cluster of genes encoding two tailocins, which significantly differs from the known tailocin-encoding clusters.

In a first aspect, the present invention thus relates to a composition comprising a cell lysate, wherein the cell produces at least one tailocin from a *Pseudomonas* bacterium selected from the group consisting of *Pseudomonas fluorescens, Pseudomonas chlororaphis, Pseudomonas koreensis* and *Pseudomonas syringae* species, for use in the prevention or the treatment in a subject of a contamination or an infection by an *Escherichia coli* strain, preferably selected from the group consisting of urogenital infection or gastrointestinal infection.

The present invention also relates to the use of a composition comprising a cell lysate, wherein the cell produces at least one tailocin from a *Pseudomonas* bacterium selected from the group consisting of *Pseudomonas fluorescens, Pseudomonas chlororaphis, Pseudomonas koreensis* and *Pseudomonas syringae* species, for the prevention and/or the treatment of a contamination by an *Escherichia coli* strain in a food product or on a non-living surface.

The present invention also relates to an intimate care product composition or a food supplement comprising a cell lysate as defined herein.

The present invention also relates to an isolated nucleic acid molecule comprising a nucleotide sequence with:
(a) at least 80% identity with SEQ ID NO:1,
(b) at least 80% identity with SEQ ID NO:2,
(c) at least 80% identity with SEQ ID NO:3, or
(d) at least 80% identity with SEQ ID NO:5.

The present invention also relates to an expression vector comprising the nucleic acid molecule according to the invention; a cell comprising a nucleic acid molecule according to the invention, or an expression vector according to the invention; and a cell lysate comprising at least one tailocin obtained from the cell according to the invention.

The present invention also relates to a method for preparing a cell lysate enriched in tailocin comprising at least the steps of:
(a) cultivating the cell according to the invention in a cell culture, preferably under conditions stimulating tailocin production by the cell, preferably tailocin production is stimulated by ultraviolet irradiation, anaerobic stress, heat induction or addition of a DNA damaging agent such as mitomycin C, ciprofloxacin, or H₂O₂ to the culture medium,
(b) obtaining a raw cell lysate by continuing the cell culture of step (a) until natural lysis of the cells or lysing the cell culture of step a) to obtain a cell lysate,
(c) filtering the cell lysate of step (b) through a first filter with a pore size between 0,1 and 0,45 µm and collecting the filtrate, and
(d) filtering the filtrate of step (c) through a second filter with a cut-off between 50 and 300 kDa and collecting the retentate, thereby obtaining a cell lysate enriched in tailocin.

The present invention also relates to a cell lysate obtained by the method for preparing a cell lysate enriched in tailocin according to the invention.

### DESCRIPTION OF THE FIGURES

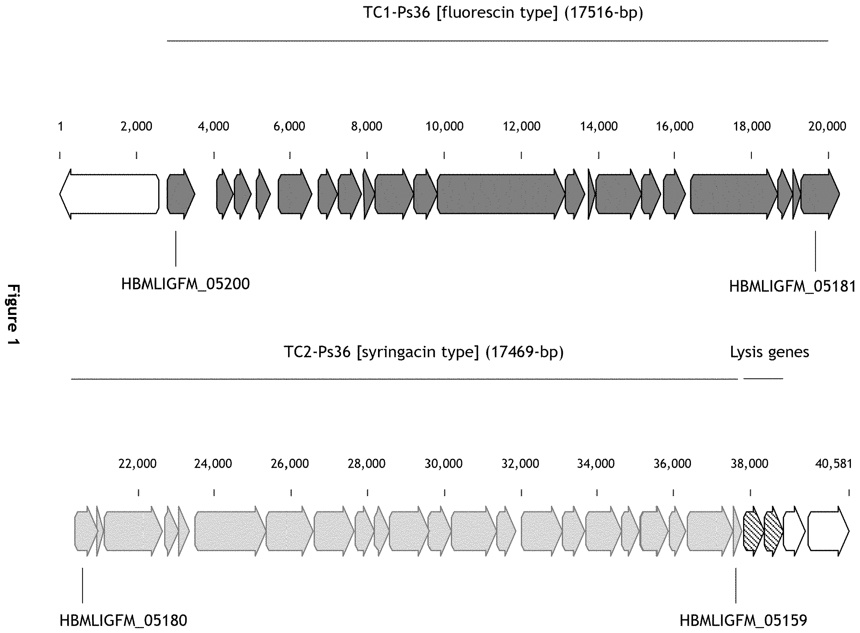

**Figure 1****. Pair of tailocin units predicted in the *Pseudomonas* Ps36 tailocin gene cluster.**

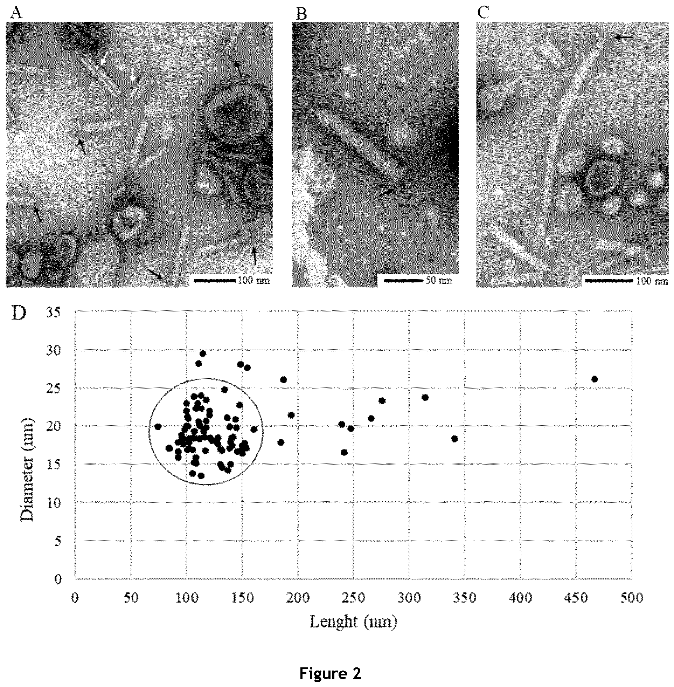

**Figure 2****. Morphology of the tailocin particles produced by *P. fluorescens* Ps36. (A-B-C)** Electron microscopy pictures of the purified tailocins. Samples were negatively stained with 2% (wt/vol) aqueous uranyl acetate. Three representative pictures are shown from these TEM preparations. Scale bar is indicated on each image. Black arrows designate tail fibers and white arrows indicate partially degraded tubes. Panel A shows the whole population of tailocins, composed in large majority by R-type particles homogeneous in term of size. Panel B shows one of the majority R-type particles. Panel C displays a long tube attached to a baseplate with associated tail fibres, characteristic to the rare irregular-length particles population. (D) Dimensions of 92 tailocins measured from TEM images. The ring highlights the majority population composed by particles of an average length and diameter of approximately 118 ± 16 nm and 19 ± 2 nm, respectively.

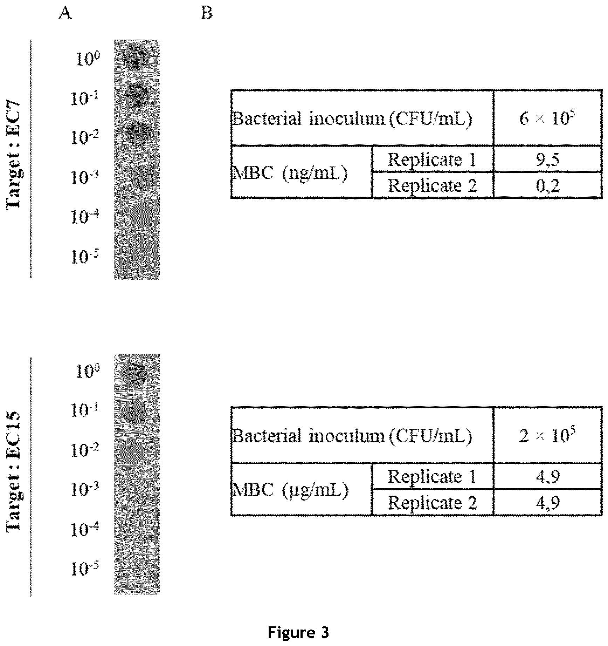

**Figure 3****. Bactericidal activity of *Pseudomonas* Ps36 purified tailocins on two strains representative of uropathogenic as well as enteropathogenic *E. coli.*** Bactericidal activities were determined by solid and liquid methods. A. Lawn assays were carried out using the overlay technique by spotting tenfold serial dilution of purified tailocin as well as the undiluted sample (2.91 mg/mL for TC-Ps36 spotted on EC7, 0.49 mg/mL for TC-Ps36 spotted on EC15). As dilutions increase, the killing zones become smaller and gradually more opaque due to partial bactericidal action. Data is representative of the two duplicates. **B.** MBC assays were performed by broth microdilution method using twofold serial dilutions starting with an undiluted purified tailocin at initial protein concentration of 10 mg/mL. MBC was determined by subculturing the broth microdilution plate in fresh medium without tailocin. Tables indicate the initial bacterial inoculum left in each well and precisely determined by CFU counting. The MBC corresponds to the lowest concentration of antibacterial agent that totally kills the initial bacterial inoculum.

### DETAILED DESCRIPTION OF THE INVENTION

In the context of the present invention, the inventors surprisingly found that a panel of tailocins produced by various *Pseudomonas* strains of the *Pseudomonas fluorescens, Pseudomonas chlororaphis, Pseudomonas koreensis* and *Pseudomonas syringae* species can unexpectedly show inter-order activity on *E. coli.* In addition, the genome of the bacterial producer showing one of the widest and strongest activities against *E. coli* was sequenced and shown to contain an original cluster of genes encoding two tailocins, which significantly differs from the known tailocin-encoding clusters.

### Definitions

The singular forms **"a," "an,"** and **"the"** are used in the sense that they include plural reference of the referenced components or steps, such as "at least one", "at least a first", "one or more" or "a plurality", unless the context clearly dictates otherwise. Thus, for example, a reference to "a cell" or "a microorganism" includes a plurality of cells or microorganisms, including mixtures thereof.

The terms **"comprise" "contain" "include"** and variations thereof such as "comprising" are used herein in an inclusive sense, i.e., to specify the presence of the stated features but meaning that any further such features can be present in various embodiments of the invention.

The terms **"consist essentially of', "consist essentially in"** and variations thereof such as "consisting essentially of" or "consisting essentially in" are used to specify the presence of the stated features but meaning that specific further such features that not materially affecting the essential characteristics of the invention can be present in various embodiments of the invention.

The terms **"consist",** and variations thereof such as "consisting of" or "consisting in" are used in an exclusive sense, i.e., to specify the presence of the stated features meaning that no further can be present in various embodiments of the invention.

The term **"about"** or **"approximately"** refers to the normal variation or range of error for a given value or range known to the person of skills in the art. It usually means a value or range within (i.e., ±) 20%, 10%, 5%, 2% or 1% of a given value or range.

As used herein, the term **"prokaryote"** refers to a single-cell microorganism whose cell structure does not include a nucleus. Prokaryotes include the kingdoms of bacteria (also called eubacteria) and archaea.

The bacterial kingdom comprises several divisions (also known as phyla), including the *Pseudomonadota* (also formerly known as Proteobacteria).

The *Pseudomonadota* division contains several classes, including the *Gammaproteobacteria* class, which in turn contains several orders, including the *Pseudomonadales* and the *Enterobacterales* orders.

The *Pseudomonadales* order contains several families, including the *Pseudomonadaceae* family, which in turn contains several genera, including the *Pseudomonas* genus, which contains several species, including the species *Pseudomonas fluorescens, Pseudomonas chlororaphis, Pseudomonas koreensis* and *Pseudomonas syringae.*

The *Enterobacterales* order contains several families, including the *Enterobacteriaceae* family, which in turn contains several genera, including the *Escherichia* genus, which contains several species, including the *Escherichia coli* species.

Among *Escherichia coli* species, strains may be classified depending on various criteria, including serotype, phylogroup or sequence type.

As used herein a bacterial **"serotype"** or **"serovar"** refers to a separate group within a bacterial species, in which all members of the group have the same antigens on their surfaces. *Escherichia coli* strains may thus be classified according to their serotype based on their major surface antigens. The two main surface antigens generally used for serotyping *Escherichia coli* strains are the O antigen (which is part of lipopolysaccharide layer and is encoded by the *rfb* gene cluster) and the H antigen (a major component of flagella, involved in *E. coli* movement, generally encoded by the *fliC* gene), and optionally the K-antigen capsule. Distinct O, H and K antigens are labelled by a number and groups of *Escherichia coli* strains can be defined based on the O and H and/or K antigens expressed by the strain. Some *Escherichia coli* serotypes are known to be particularly associated to gastrointestinal *E. coli* infections. Those include but are not limited to the 0157, 026, 045, 0103, O111, 0121, and 0145 serotypes. Particular examples of serotypes known to be particularly associated to gastrointestinal *E. coli* infections and for which the inventors showed bactericidal activity of the tested tailocins include O157:H7, O44:H18, O111:K58(B4) and O26:H11 serotypes (Alharbi et al., 2022, Bumunang et al., 2023) .

As used herein a bacterial **"phylogroup"** or **"phylogenetic group"** refers to a group comprising bacterial strains that share a common ancestor. Phylogroup classification is thus based on evolutionary history rather than expressed surface antigens. *E. coli* strains can be divided into seven main phylogroups termed A, B (including subgroups B1, B2), C, D, E, F and G. Phylogroups B2 and D are particularly associated with urinary tract infections (Kot, 2019).

As used herein a bacterial **"sequence type"** or **"ST"** refers to a classification of bacterial strains based on multilocus sequence typing (MLST). For *E. coli,* sequence typing is according to Tartof et al. 2005. The ST of a particular *E. coli* strain can be determined based on its genomic sequence using the PubMLST public database available at https://pubmlst.org/. *E. coli* ST131 is known to be particularly associated with multidrug-resistant urinary tract infections (Lavigne et al., 2012, Nicolas-Chanoine et al., 2014).

As used herein, a **"contamination"** by an *E. coli* strain in a subject or a product or on a non-living surface refers to the presence of this *E. coli* strain in the subject or the product or on the non-living surface. In the case of a subject, a contamination refers to the preliminary phase before any symptom is present in the subject.

As used herein, an **"infection"** by an *E*. coli strain in a subject refers to the presence of this *E. coli* strain in the subject, associated to symptoms, i.e. a physical feature which is regarded as indicating a condition of disease. In the case of urinary *E. coli* infection, symptoms include, i) for cystitis: an infection of the bladder with accompanying symptoms of dysuria (painful urination), pain (particularly suprapubic), urinary frequency, urinary urgency and hematuria (blood in urine), ii) for pyelonephritis: a kidney infection characterized by cystitis symptoms with additional fever, flank pain, costovertebral-angle tenderness, nausea and vomiting (Flores-Mireles et al., 2015). In the case of gastrointestinal *E. coli* infection, symptoms include mild watery diarrhea to bloody diarrhea (hemorrhagic colitis) accompanied by fever, abdominal cramping, or vomiting (Croxen et al., 2013)

As used herein, a bacterium or cell **"lysate"** refers to a raw composition comprising a population of a cell (including a bacterium), a proportion of which have disrupted membranes (it will then be referred to as a **"raw lysate**"), or a purified composition derived by purification of a raw lysate (it will then be referred to as a **"purified lysate**"). For instance, a purified lysate includes a lysate in which the debris from bacterial or cell membranes have been removed by filtration. Such filtration will generally also remove possibly alive microorganisms, including non-lysed bacteria or cells. A purified lysate further includes a lysate that has been enriched in specific constituents, and in particular a lysate that has been enriched in tailocins produced by the lysed bacterium or cell.

As used herein, a **"bacteriocin"** refers to a proteinaceous or peptidic compound produced by one or more strains of bacteria and active against (i.e. inhibiting the growth of) some other bacterial strains.

As used herein, a **"tailocin"** or "phage tail-like bacteriocin" refers to high-molecular-weight bacteriocins, encoded by bacterial genomes, showing structural similarities with bacteriophage tails. They consist of a contractile (R-type) or non-contractile (F-type) sheath forming a rigid tube attached to a baseplate with a spike-shaped protein complex at its tip and associated receptor binding protein (RBP). These nanomachines kill upon contact with their targeted bacteria probably by puncturing their membranes leading to cell lysis (Ghequire and Mot, 2015). Their specificity is conferred by their receptor-binding proteins (RBP) on the tailocin tail fibers and the cell surface receptors of the target bacterium, notably the lipopolysaccharide (LPS). In addition to classification based on their contractile (R-type) or non-contractile (F-type) sheath, tailocin may also be classified as fluorescin type or syringacin type, depending on their sequence and the organism from which they were firstly identified (fluorescin-type tailocins are those with high sequence identity to the tailocin of *Pseudomonas fluorescens,* while syringacin-type tailocins are those with the tailocin of *Pseudomonas syringae,* as described in Ghequire and Mot, 2015).

As used herein, **"episomal vectors"** or **"episomes"** are free, circular, self-replicating extrachromosomal DNA molecules of viral and nonviral origin that replicate in both eukaryotic and prokaryotic cells. Viral episomes refer to viruses whose natural life cycle includes a stage of remaining as free, viral DNA within the cell nucleus, retaining the ability to encode proteins without integrating into the host cell's genome. Examples include Adenoviruses, a family of DNA viruses, or the adeno-associated virus (AAV), a small, nonpathogenic satellite virus. Nonviral episomes are plasmids, usually of a larger size relative to conventional plasmids, and can exist independent of the genomic DNA for longer periods than conventional plasmids, due to an origin of replication (OriPs) that derives from viral genomes, such as those of human papovavirus BK, bovine papillomavirus type 1, Epstein-Barr virus (EBV), and simian virus 40 (SV40), along with sequences that support retention of the episomes in the host cell (referred to as a vector maintenance sequence). The term **"origin of replication"** refers to a virus DNA sequence at which nucleic acid, typically DNA, synthesis is initiated. The term **"vector maintenance sequence"** refers to a cis-acting nucleic acid sequence contained in a vector and which confers long term stable vector replication to the vector, permitting to the vector to remain unrearranged and stably maintained at a constant copy number. Viral derived vector maintenance sequences are known in the art.

As used herein, a **"Bacterial Artificial Chromosome"** (abbreviated as "**BAC**") refers to an engineered DNA construct used to clone DNA sequences in bacterial cells based on a functional fertility plasmid (also referred to as an « F-plasmid"), which contains partition genes that promote the even distribution of plasmids after bacterial cell division. The two main components of a BAC include:
- A selectable marker gene which allows for the identification of successful transformants; and
- An origin of replication which triggers DNA replication within the bacterial cell. While an average plasmid can only carry up to 20kb of foreign DNA sequence, a BAC can carry between 150-350kb.

As used herein, the term **"therapy"** refers to any protocol, method and/or agent (in particular a compound, i.e., a "therapeutic compound") that can be used in the prevention, management, treatment and/or amelioration of a disease, disorder, or condition in a patient. The patient may be at risk of contracting the disease, disorder, or condition, or suspected to have contracted the disease, disorder, or condition. Alternatively, the patient may have been diagnosed as suffering from the disease, disorder or condition. Non-limiting examples of therapy comprise administration of a composition (e.g., a pharmaceutical or a vaccinal composition), physical therapy (e.g., radiotherapy, ultrasound therapy, electrotherapy, phototherapy, cryotherapy, etc.), physiotherapy, psychological therapy, etc. Therapy also includes the possibility to use combination therapy. "Combination therapy" and any variation thereof such as "combined use" refers to the action of delivering to the same subject several distinct therapies. Such a combination encompasses the cases where the distinct therapies are delivered to the subject as a single composition (together in the same unit dosage) or separately (i.e. dissociate arrangement), in which case the distinct therapies may be delivered simultaneously or sequentially. "Simultaneously", "concomitantly" or "concurrently", refers to the action of delivering essentially at the same time or over the same period of time (e.g., within one hour or less) several therapies either mixed in the same composition or separately. "Sequentially" refers to "one after the other", meaning one therapy being delivered first followed, immediately or after a suitable period of time, by the delivery of another therapy.

When therapy comprises administering or the administration of a composition, the composition is administered in an amount, manner, and/or mode effective to treat or prevent the patient's disease, disorder, or condition. Therapy may require administration of the composition more than once. In such case, the intervals between two successive deliveries of the therapy may be identical or different. In case of combination therapy, the intervals between two successive deliveries of the several distinct therapies may be identical or different.

As used herein, the term **"treating"** or **"treatment"** means an improvement of the patient's disease, disorder, or condition, which may be observed at the clinical, histological, and/or biochemical level. The terms "treating" or "treatment" notably include improving a clinical, histological, and/or biochemical symptom or parameter associated with the patient's disease, disorder, or condition or inhibiting, reducing, or delaying progression or exacerbation of the patient's disease, disorder, or condition (including secondary damage caused by the disease, disorder, or condition) to either a statistically significant degree or to a degree detectable to one skilled in the art. In some embodiments, treatment is assessed on a population basis such that a therapy is considered to "treat" a particular disease, disorder or condition if a statistically significant improvement of the patient's disease, disorder, or condition is observed in a population suffering from the disease, disorder, or condition.

As used herein, the terms **"prevent", "prevention"** and **"preventing"** refer to the reduction in the risk for a subject of acquiring or developing a given disease, disorder, or condition. The terms "prevent", "prevention" and "preventing" also comprise delaying the onset and/or reducing the frequency and/or intensity of clinical, histological and/or biochemical symptoms or parameters associated with this given disease, disorder, or condition. In some embodiments, prevention is assessed on a population basis such that a therapy is considered to "prevent" a particular disease, disorder or condition if a statistically significant decrease in the risk of acquiring or developing the disease, disorder, or condition, and/or a statistically significant delay of the onset and/or a statistically significant decrease of the frequency and/or intensity of clinical, histological and/or biochemical symptoms or parameters associated with the disease, disorder or condition, is observed in a population susceptible to the disease, disorder, or condition. The term **"therapeutically effective amount"** refers to an amount effective of a therapeutic agent, at dosages and for periods of time necessary, to elicit the desired biological response in a subject. In other words, the therapeutic effective amount is the amount of the therapeutic agent which is sufficient to treat or prevent a given disease, disorder or condition and/or a symptom related thereto. The therapeutical effective amount will be adjusted to the individual requirements in each particular case. It can vary within wide limits depending upon numerous factors such as the nature and the severity of the disease, disorder or condition to be treated, the age and general health condition of the patient, other medicaments with which the patient is being treated, the route and form of administration and the preferences and experience of the medical practitioner involved. A therapeutically effective amount is also one in which any toxic or detrimental effects of the agent are outweighed by the therapeutically beneficial effects.

As used herein, a **"subject"** may be any animal, in particular any mammalian animal including humans as well as pets and livestock, including dog, cat, cattle, goat, pig, swine, sheep and monkey. A human subject suffering from a disease or suspected of suffering from disease or diagnosed with a disease may be designated as a patient.

The **percent identities** referred to in the context of the disclosure of the present invention are determined on the after optimal alignment of the sequences to be compared, which may therefore comprise one or more insertions, deletions, truncations and/or substitutions.

This percent identity may be calculated by any sequence analysis method well-known to the person skilled in the art.

The percent identity may be determined after global alignment of the sequences to be compared of the sequences taken in their entirety over their entire length. In addition to manual comparison, it is possible to determine global alignment using the algorithm of Needleman and Wunsch (1970).

For nucleotide sequences, the sequence comparison may be performed using any software well-known to a person skilled in the art, such as the Needle software. The parameters used may notably be the following: "Gap open" equal to 10.0, "Gap extend" equal to 0.5, and the EDNAFULL matrix (NCBI EMBOSS Version NUC4.4).

For amino acid sequences, the sequence comparison may be performed using any software well-known to a person skilled in the art, such as the Needle software. The parameters used may notably be the following: "Gap open" equal to 10.0, "Gap extend" equal to 0.5, and the BLOSUM62 matrix.

Preferably, the percent identify as defined in the context of the present invention is determined via the global alignment of sequences compared over their entire length.

### Therapeutic uses of a Pseudomonas fluorescens, Pseudomonas chlororaphis, Pseudomonas koreensis or Pseudomonas syringae lysate comprising at least one tailocin against contamination or infection by an Escherichia coli strain

In a first aspect, the present invention relates to a composition (preferably a pharmaceutical composition) comprising a cell lysate, wherein the cell produces at least one tailocin from a *Pseudomonas* bacterium selected from the group consisting of *Pseudomonas fluorescens, Pseudomonas chlororaphis, Pseudomonas koreensis* and *Pseudomonas syringae* species, for use in the prevention or the treatment in a subject of a contamination or an infection by an *Escherichia coli* strain, preferably selected from the group consisting of urogenital infection or gastrointestinal infection.

The present invention also relates to the use of a composition comprising a cell lysate, wherein the cell produces at least one tailocin from a *Pseudomonas* bacterium selected from the group consisting of *Pseudomonas fluorescens, Pseudomonas chlororaphis, Pseudomonas koreensis* and *Pseudomonas syringae* species, for manufacturing a drug for use in the prevention or the treatment in a subject of a contamination or an infection by an *Escherichia coli* strain, preferably selected from the group consisting of urogenital infection or gastrointestinal infection.

The present invention also relates to the use of a composition comprising a cell lysate, wherein the cell produces at least one tailocin from a *Pseudomonas* bacterium selected from the group consisting of *Pseudomonas fluorescens, Pseudomonas chlororaphis, Pseudomonas koreensis* and *Pseudomonas syringae* species, for the prevention or the treatment in a subject of a contamination or an infection by an *Escherichia coli* strain, preferably selected from the group consisting of urogenital infection or gastrointestinal infection.

The present invention also relates to a method for preventing or treating in a subject in need thereof a contamination or an infection by an *Escherichia coli* strain, preferably selected from the group consisting of urogenital infection or gastrointestinal infection, comprising administering to the subject a therapeutically efficient amount of a composition comprising a cell lysate, wherein the cell produces at least one tailocin from a *Pseudomonas* bacterium selected from the group consisting of *Pseudomonas fluorescens, Pseudomonas chlororaphis, Pseudomonas koreensis* and *Pseudomonas syringae* species.

### Cell and tailocin

The inventors have shown that bacterial lysates from various *Pseudomonas fluorescens, Pseudomonas chlororaphis, Pseudomonas koreensis* and *Pseudomonas syringae* species encode tailocins that unexpectedly display bactericidal activity against *E. coli,* even though *E. coli* does not belong to the same species, nor to the same genus, the same family or even the same order. In fact, the *Pseudomonas* genus belongs to an order (*Pseudomonadales*) different from the order to which belongs *Escherichia* genus (*Enterobacterales*)*.*

On this basis, the composition used in the invention can comprise any cell lysate, provided that the cell produces at least one tailocin from a *Pseudomonas* bacterium selected from the group consisting of *Pseudomonas fluorescens, Pseudomonas chlororaphis, Pseudomonas koreensis* and *Pseudomonas syringae* species.

In a preferred embodiment, the cell lysate is a bacterial lysate from any *Pseudomonas fluorescens, Pseudomonas chlororaphis, Pseudomonas koreensis* and *Pseudomonas syringae* species that produces at least one tailocin.

Preferred *Pseudomonas fluorescens, Pseudomonas chlororaphis, Pseudomonas koreensis* and *Pseudomonas syringae* strains that may be used for preparing the bacterial lysate are those disclosed herein as encoding tailocins that display bactericidal activity against *E*. *coli* and are described in **Table 1** below.

**Table 1. Preferred Pseudomonas fluorescens, Pseudomonas chlororaphis, Pseudomonas koreensis and Pseudomonas syringae strains that may be used for preparing the bacterial lysate comprised in the composition for use according to the invention.**

| Species | Strain | Collection (accession number and optional deposit date) | Sequence identifier or Genbank accession number* of the tailocin cluster(s) |
|---|---|---|---|
| *P. fluorescens* | Ps36 | CNCM (I-6068, deposited on the 21^{st} of March 2024)** | SEQ ID NO:1 (cluster encoding a fluorescin type tailocin), |
| | | | SEQ ID NO:2 (cluster encoding a syringacin type tailocin), |
| | | | SEQ ID NO:3 (both clusters) |
| | | | SEQ ID NO:4 (lysis genes associated to the cluster) |
| | | | SEQ ID NO:5 (both clusters and lysis genes associated to the cluster) |
| *P. fluorescens* | Ps40 | CFBP (3353) | N.D. |
| *P. fluorescens* | Ps41 | CFBP (4560) | N.D. |
| *P. fluorescens* | Ps45 | CFBP (5900) | N.D. |
| *P. fluorescens* | Ps46 | CFBP (5916) | N.D. |
| *P. chlororaphis subsp. piscium* | Ps54 | DSMZ (DSM 21509) | Genbank NZ_CP027707.1 (region 1361167-1399480) |
| *P. chlororaphis subsp. chlororaphis* | Ps38 | DSMZ (DSM 50083) | N.D. |
| *P. chlororaphis subsp. aureofaciens* | Ps53 | DSMZ (DSM 6698) | N.D. |
| *P. chlororaphis* | Ps6 | CNCM (I-6066, deposited on the 21^{st} of March 2024)** | N.D. |
| *P. syringae pv aptata* | S18 | CFBP 5471 | N.D. |
| *P. syringae pv aptata* | S19 | CFBP 5468 | N.D. |
| *P. syringae pv aptata* | S24 | CFBP 3313 | N.D. |
| *P. syringae pv aptata* | S37 | CFBP 5429 | N.D. |
| *P. koreensis* | Ps4 | CNCM (I-6065, deposited on the 21^{st} of March 2024)** | N.D. |
| *P. koreensis* | Ps7 | CNCM (I-6067, deposited on the 21^{st} of March 2024)** | N.D. |

| | | | |
|---|---|---|---|
| CNCM: Collection nationale de cultures de microorganismes (Institut Pasteur, 25-28 rue du Docteur Roux, 75724 Paris Cedex 15, France, https://www.pasteur.fr/en/cncm); CFBP: Collection française des bactéries associées aux plantes (CIRM-CFBP, INRAE - IRHS, 42 rue Georges Morel - CS 60057, 49071 Beaucouzé cedex - France, https://cirm-cfbp.fr/); DSMZ: Deutsche Sammlung von Mikroorganismen und Zellkulturen GmbH (Inhoffenstraße 7B, 38124 Braunschweig, Science Campus Braunschweig-Süd, GERMANY, https://www.dsmz.de/). *Genbank release 258 of October 15, 2023. **Deposit under Budapest treaty provisions. N.D.: Not determined. | | | |

Further *Pseudomonas fluorescens, Pseudomonas chlororaphis, Pseudomonas koreensis* and *Pseudomonas syringae* strains that may be used for preparing the bacterial lysate are genetically engineered variants of any one of the strains of **Table 1** above, in particular genetically engineered variants with stronger expression of the tailocin gene cluster, such as those in which the promoter controlling the expression of the tailocin cluster has been replaced by a stronger promoter or the ribosome binding site (RBS) has been replaced by a stronger RBS. Indeed, replacing the native promoter by a stronger homologous (i.e. another promoter presents in the genome of the same strain) or heterologous (i.e. a promoter from another species) promoter or replacing the native RBS by a stronger RBS may improve tailocin production by the genetically engineered variant and thus the concentration of tailocin in the lysate. However, genetic variants of any one of the strains of **Table 1** above with other modifications (e.g. metabolic pathways modulation, cofactors modulation, inactivation of repressors, etc.) resulting in higher production of the tailocin(s) are also part of the present invention.

In addition, other *Pseudomonas fluorescens, Pseudomonas chlororaphis, Pseudomonas koreensis* and *Pseudomonas syringae* strains that may be used for preparing the bacterial lysate are those which genome (either intrachromosomal or extrachromosomal) comprises a cluster encoding a tailocin that is closely related to a tailocin encoded by the genome of the strains of **Table 1** above.

As used herein, a candidate tailocin will be considered as closely related to another reference tailocin when its genome comprises a cluster encoding a tailocin with a nucleotide sequence having at least 80%, preferably at least 85%, more preferably at least 90%, at least 91%, at least 92%, at least 93%, at least 94%, at least 95%, at least 96%, at least 97%, at least 98%, at least 99% or even at least 99.5% identity with the nucleotide sequence of a cluster encoding the reference tailocin. Preferably, the nucleotide sequence of the cluster encoding a closely related candidate tailocin will further encode proteins with amino acid sequences having at least 80%, preferably at least 85%, more preferably at least 90%, at least 91%, at least 92%, at least 93%, at least 94%, at least 95%, at least 96%, at least 97%, at least 98%, at least 99% or even at least 99.5% identity with the amino acid sequences of corresponding proteins encoded by the nucleotide sequence of the cluster encoding the reference tailocin. Notably, the nucleotide sequence of the cluster encoding a closely related candidate tailocin will preferably have at least 80% identity with the nucleotide sequence of a cluster encoding the reference tailocin and encode proteins with amino acid sequences having at least 80% identity with the amino acid sequences of corresponding proteins encoded by the nucleotide sequence of a cluster encoding the reference tailocin. More preferably, the nucleotide sequence of the cluster encoding a closely related candidate tailocin will preferably have at least 90% identity with the nucleotide sequence of a cluster encoding the reference tailocin and encode proteins with amino acid sequences having at least 90% identity with the amino acid sequences of corresponding proteins encoded by the nucleotide sequence of a cluster encoding the reference tailocin. Even more preferably, the nucleotide sequence of the cluster encoding a closely related candidate tailocin will preferably have at least 95% identity with the nucleotide sequence of a cluster encoding the reference tailocin and encode proteins with amino acid sequences having at least 95% identity with the amino acid sequences of corresponding proteins encoded by the nucleotide sequence of a cluster encoding the reference tailocin.

In particular, other *Pseudomonas fluorescens, Pseudomonas chlororaphis, Pseudomonas koreensis* and *Pseudomonas syringae* strains that may be used for preparing the bacterial lysate are those which genome comprises a cluster encoding a tailocin with a nucleotide sequence having at least 80%, preferably at least 85%, more preferably at least 90%, at least 91%, at least 92%, at least 93%, at least 94%, at least 95%, at least 96%, at least 97%, at least 98%, at least 99% or even at least 99.5% identity with SEQ ID NO:1 (the cluster of *P. fluorescens* Ps36 encoding a fluorescin type tailocin), SEQ ID NO:2 (the cluster of *P. fluorescens* Ps36 encoding a syringacin type tailocin), SEQ ID NO:3 (which comprises both SEQ ID NO:1 and SEQ ID NO:2), or even SEQ ID NO:5 (which comprises SEQ ID NO:3 and the lysis genes associated to the cluster, i.e. SEQ ID NO:4). Preferably, the nucleotide sequence of the tailocin-encoding cluster further encodes proteins with amino acid sequences having at least 80%, preferably at least 85%, more preferably at least 90%, at least 91%, at least 92%, at least 93%, at least 94%, at least 95%, at least 96%, at least 97%, at least 98%, at least 99% or even at least 99.5% identity with the amino acid sequences of corresponding proteins encoded by SEQ ID NO:1, SEQ ID NO:2, SEQ ID NO:3 or SEQ ID NO:5, respectively. Notably, preferred other *Pseudomonas fluorescens, Pseudomonas chlororaphis, Pseudomonas koreensis* and *Pseudomonas syringae* strains that may be used for preparing the bacterial lysate are those which genome comprises a cluster encoding a tailocin:
- with a nucleotide sequence having at least 80% identity with SEQ ID NO:1 and encoding proteins with amino acid sequences having at least 80% identity with the amino acid sequences of corresponding proteins encoded by SEQ ID NO:1, preferably with a nucleotide sequence having at least 90% identity with SEQ ID NO:1 and encoding proteins with amino acid sequences having at least 90% identity with the amino acid sequences of corresponding proteins encoded by SEQ ID NO:1, more preferably with a nucleotide sequence having at least 95% identity with SEQ ID NO:1 and encoding proteins with amino acid sequences having at least 95% identity with the amino acid sequences of corresponding proteins encoded by SEQ ID NO:1,
- with a nucleotide sequence having at least 80% identity with SEQ ID NO:2 and encoding proteins with amino acid sequences having at least 80% identity with the amino acid sequences of corresponding proteins encoded by SEQ ID NO:2, preferably with a nucleotide sequence having at least 90% identity with SEQ ID NO:2 and encoding proteins with amino acid sequences having at least 90% identity with the amino acid sequences of corresponding proteins encoded by SEQ ID NO:2, more preferably with a nucleotide sequence having at least 95% identity with SEQ ID NO:2 and encoding proteins with amino acid sequences having at least 95% identity with the amino acid sequences of corresponding proteins encoded by SEQ ID NO:2,

- with a nucleotide sequence having at least 80% identity with SEQ ID NO:3 and encoding proteins with amino acid sequences having at least 80% identity with the amino acid sequences of corresponding proteins encoded by SEQ ID NO:3, preferably with a nucleotide sequence having at least 90% identity with SEQ ID NO:3 and encoding proteins with amino acid sequences having at least 90% identity with the amino acid sequences of corresponding proteins encoded by SEQ ID NO:3, more preferably with a nucleotide sequence having at least 95% identity with SEQ ID NO:3 and encoding proteins with amino acid sequences having at least 95% identity with the amino acid sequences of corresponding proteins encoded by SEQ ID NO:3, or
- with a nucleotide sequence having at least 80% identity with SEQ ID NO:5 and encoding proteins with amino acid sequences having at least 80% identity with the amino acid sequences of corresponding proteins encoded by SEQ ID NO:5, preferably with a nucleotide sequence having at least 90% identity with SEQ ID NO:5 and encoding proteins with amino acid sequences having at least 90% identity with the amino acid sequences of corresponding proteins encoded by SEQ ID NO:5, more preferably with a nucleotide sequence having at least 95% identity with SEQ ID NO:5 and encoding proteins with amino acid sequences having at least 95% identity with the amino acid sequences of corresponding proteins encoded by SEQ ID NO:5.

*Pseudomonas fluorescens, Pseudomonas chlororaphis, Pseudomonas koreensis* and *Pseudomonas syringae* strains comprising a gene cluster encoding a tailocin that is closely related to a tailocin encoded by the genome of the strains of Table 1 above, and which shows improved bactericidal activity (either by lower Minimum Bactericidal Concentration (MBC) or by broader host range), in particular against *E. coli,* are particularly preferred.

Regarding *Pseudomonas fluorescens, Pseudomonas chlororaphis, Pseudomonas koreensis* and *Pseudomonas syringae* strains of **Table 1** other than *Pseudomonas fluorescens* strain Ps36, a skilled person will know how to sequence the genome of these strains, identify the cluster(s) encoding tailocin(s), and compare it (nucleotide sequence and optionally amino acid sequences of encoded proteins) to any other cluster encoding a tailocin from another strain of the *Pseudomonas fluorescens, Pseudomonas chlororaphis, Pseudomonas koreensis* and *Pseudomonas syringae* species. Therefore, other *Pseudomonas fluorescens, Pseudomonas chlororaphis, Pseudomonas koreensis* and *Pseudomonas syringae* strains that may be used for preparing the bacterial lysate are those which genome comprises a cluster encoding a tailocin that is closely related to (as defined above) a tailocin encoded by the genome of any of the strains of **Table 1** above.

Finally, as tailocins from several strains of the *Pseudomonas fluorescens, Pseudomonas chlororaphis, Pseudomonas koreensis* and *Pseudomonas syringae* species have been shown to have bactericidal activity against several *E. coli* strains, a skilled person would expect that tailocins encoded by most strains of the *Pseudomonas fluorescens, Pseudomonas chlororaphis, Pseudomonas koreensis* and *Pseudomonas syringae* species would also show at least some bactericidal activity against one or more *E. coli* strains.

Therefore, *Pseudomonas fluorescens, Pseudomonas chlororaphis, Pseudomonas koreensis* and *Pseudomonas syringae* strains encoding known tailocin clusters can also be used for preparing the bacterial lysate comprised in the composition for use according to the invention. In particular, *Pseudomonas fluorescens, Pseudomonas chlororaphis, Pseudomonas koreensis* and *Pseudomonas syringae* strains comprising (natively or by genetic engineering) one of the tailocin clusters presented in Table 2 below, or a closely related tailocin (as defined above, i.e. with a tailocin-encoding cluster with at least 80%, preferably at least 85%, more preferably at least 90%, at least 91%, at least 92%, at least 93%, at least 94%, at least 95%, at least 96%, at least 97%, at least 98%, at least 99% or even at least 99.5% identity with the nucleotide sequence of a tailocin-encoding cluster presented in **Table 2,** and optionally encoding proteins with amino acid sequences having at least 80%, preferably at least 85%, more preferably at least 90%, at least 91%, at least 92%, at least 93%, at least 94%, at least 95%, at least 96%, at least 97%, at least 98%, at least 99% or even at least 99.5% identity with the amino acid sequences of corresponding proteins encoded by the tailocin-encoding cluster presented in **Table 2**) can also be used for preparing the bacterial lysate comprised in the composition for use according to the invention.

**Table 2. Known tailocin clusters from Pseudomonas fluorescens, Pseudomonas chlororaphis, or Pseudomonas syringae strains.**

| Species | Strain | Tailocin type | Genbank accession number* (region) | Region in Genbank sequence |
|---|---|---|---|---|
| *P. fluorescens* | SF4c | fluorescin | NZ_JTGH01000018 | 4056-20401 |
| *P. fluorescens* | SF4c | syringacin | NZ_JTGH01000018 | 20321-37466 |
| *P. fluorescens* | Pf0-1 | fluorescin | NC_007492.2 | 1313085-1328442 |
| *P. fluorescens* | Pf0-1 | syringacin | NC_007492.2 | 1328428-1344539 |
| *P. fluorescens* | SBW25 | fluorescin | NC_012660.1 | 1303690-1316657 |
| *P. fluorescens* | B728a | syringacin | CP000075.1 | 5444061-5457767 |
| *P. chlororaphis subsp. chlororaphis* | LMG5004 | N.D. | NZ_LHVC00000002.1 | 513532-549874 |
| *P. chlororaphis subsp. aureofaciens* | LMG1245 | N.D. | NZ_LHVA00000010.1 | 205699-242376 |
| *P. chlororaphis subsp. chlororaphis* | 30-84 | N.D. | NZ_CM001559.1 | 1516735-1554952 |
| *P. chlororaphis subsp. piscium* | PCL1391 | N.D. | NZ_CP027736.1 | 1333239-1350234 |
| *P. chlororaphis subsp. aureofaciens* | R47 | N.D. | CP019399.1 | 1365015-1402568 |
| *P. chlororaphis subsp. aureofaciens* | CD | N.D. | NZ_LHVB00000005.1 | 564152-601358 |
| *P. chlororaphis subsp. aurantiaca* | JD37 | N.D. | NZ_CP009290.1 | 5263096-5300712 |
| *P. fluorescens* | FW300-N2C3 | N.D. | CP012831.1 | 1267205-1303726 |
| *P. fluorescens* | FW300-N2E2 | N.D. | NZ_CP015225.1 | 3484907-3520802 |
| *P. fluorescens* | FW300-N2F2 | N.D. | NZ_JACNDQ010000003 | 1198163-1236410 |

| | | | | |
|---|---|---|---|---|
| * Genbank release 258 of October 15, 2023. N.D.: Not determined. | | | | |

Finally, any other *Pseudomonas fluorescens, Pseudomonas chlororaphis, Pseudomonas koreensis* and *Pseudomonas syringae* strain producing at least one tailocin may be used. Such strains may be identified either by various methods, including:
- identifying a gene cluster encoding one or more tailocin(s) through genome sequencing and analysis,
- microscopy analysis of a bacterial lysate, or
- using functional tests on a set of target *E. coli* strains.

Regarding genome sequencing and analysis, the R- and F-type tailocin gene clusters are frequently found between the *trpE* and *trpG* genes, or between the *mutS* and *recA*/*cinA* genes in Pseudomonas (Ghequire and Mot, 2015). Therefore, after genome sequencing and annotation, these regions can be analysed for the presence of one or more gene cluster(s) encoding tailocins. Other regions containing predicted prophage sequences (for instance using the PHASTER tool) may also be analysed. Among the targeted genome regions, tailocin-encoding clusters can be identified by the presence of open-reading frames (ORF) encoding proteins with predicted functions characteristic of tailocin gene clusters (i.e. tail assembly, regulation, lysis) and lacking genes involved in capsid formation. An example of identification of a gene cluster encoding two tailocins in *Pseudomonas fluorescens* strain Ps36 is described in Example 1.

Regarding microscopy analysis of a bacterial lysate, tailocins produced by a strain can be detected by transmission electron microscopy (TEM). Indeed, tailocins have a characteristic shape (a contractile (R-type) or non-contractile (F-type) sheath forming a rigid tube attached to a baseplate) that may be clearly identified by transmission electron microscopy (TEM), the non-contracted sheath having a size in the longest direction that may be from about 50 nm to about 500 nm, while the shorter direction has a size from about 10 nm to about 35 nm. A specific example of identification of two populations of tailocins produced by *Pseudomonas fluorescens* strain Ps36 is described in Example 1.

Finally, *Pseudomonas fluorescens, Pseudomonas chlororaphis, Pseudomonas koreensis* and *Pseudomonas syringae* strains of interest may also be characterized directly by testing the desired bactericidal activity on a set of target *E. coli* strains. Bacterial lysates may notably be screened semi-quantitatively by a dilution method, in which serial dilutions of purified lysates are spotted onto bacterial lawns of target *E. coli* strains. Bactericidal activity of tailocins can then be detected based on the zone of clearing on the *E. coli* bacterial lawn. A specific example of screening of bacterial lysates from *Pseudomonas fluorescens, Pseudomonas chlororaphis,* and *Pseudomonas syringae* strains is described in Example 1. Bacterial lysates may also be screened by using the gold standard method of MBC, in which serial dilutions of the bacterial lysate are added to a defined quantity of target *E. coli* strains in liquid medium. After incubation and plating of each mixture, the absence of *E. coli* growth demonstrates the bactericidal activity of the bacterial lysate.

The tailocin produced by the *Pseudomonas fluorescens, Pseudomonas chlororaphis, Pseudomonas koreensis* or *Pseudomonas syringae* strain has preferably been confirmed to show bactericidal activity on a set of target *E. coli* strains.

In another embodiment, the cell may be a recombinant cell that has been engineered to produce at least one tailocin from a *Pseudomonas* bacterium selected from the group consisting of *Pseudomonas fluorescens, Pseudomonas chlororaphis, Pseudomonas koreensis* and *Pseudomonas syringae* species.

The recombinant cell has preferably been engineered to contain a nucleic acid molecule or a vector comprising a gene cluster encoding one or more tailocin(s) from a *Pseudomonas* bacterium selected from the group consisting of *Pseudomonas fluorescens, Pseudomonas chlororaphis, Pseudomonas koreensis* and *Pseudomonas syringae* species.

Any appropriate vector may be used, depending on the type of selected recombinant cell. When the selected recombinant cell is a bacterium, the vector is preferably selected from an episomal vector and a Bacterial Artificial Chromosome (BAC). Episomal vectors can also be used in eukaryotic cells and may thus be used when the selected recombinant cell is a eukaryotic cell.

Examples of suitable episomal vectors include T7 (Studier, 1990), pMAL, pGEX, pTRC, pBAD (Guzman, 1995), and pET (Mierendorf, 1998) vectors.

Examples of suitable BACs include pSmartBAC-S (Plasmid reference on Addgene : #107268), pCAP05 (Plasmid reference on Addgene : #102920), and pCAP-BAC (Plasmid reference on Addgene : #120229).

Any cell able to recombinantly produce one or more tailocin(s) from a *Pseudomonas* bacterium selected from the group consisting of *Pseudomonas fluorescens, Pseudomonas chlororaphis, Pseudomonas koreensis* and *Pseudomonas syringae* species may be used as host cell.

Bacteria, and more particularly *Pseudomonas* strains from the *Pseudomonas fluorescens, Pseudomonas chlororaphis, Pseudomonas koreensis* and *Pseudomonas syringae* species, are particularly suitable host cells.

However, eukaryotic cells may also be used.

### Cell lysate

The cell lysate of the composition for use according to the invention may be a raw cell lysate or preferably a purified cell lysate, which is preferably enriched in tailocin.

### Method for preparing a purified cell lysate enriched in tailocin

While any suitable method may be used, such a purified cell lysate enriched in tailocin may preferably be obtained by a method comprising at least the steps of:
(a) cultivating the cell (i.e. the cell producing a tailocin from a *Pseudomonas* bacterium selected from the group consisting of *Pseudomonas fluorescens, Pseudomonas chlororaphis, Pseudomonas koreensis* and *Pseudomonas syringae* species) in a cell culture,
(b) obtaining a raw cell lysate by continuing the cell culture of step (a) until natural lysis of the cells or lysing the cell culture of step (a),
(c) filtering the raw cell lysate of step (b) through a first filter with a pore size between 0,1 and 0,45 µm and collecting the filtrate, and
(d) filtering the filtrate of step (c) through a second filter with a cut-off between 50 and 300 kDa and collecting the retentate, thereby obtaining a purified cell lysate enriched in tailocin.

### Step (a)

In step (a), the cell producing a tailocin from a *Pseudomonas* bacterium selected from the group consisting of *Pseudomonas fluorescens, Pseudomonas chlororaphis, Pseudomonas koreensis* and *Pseudomonas syringae* species (preferably the *Pseudomonas* bacterium selected from the *Pseudomonas fluorescens, Pseudomonas chlororaphis, Pseudomonas koreensis* and *Pseudomonas syringae* species) is cultivated in an appropriate culture medium and under appropriate conditions for the cell to produce the tailocin.

Any culture medium appropriate for the culture of the selected cell may be used.

When using a *Pseudomonas* bacteria selected from the *Pseudomonas fluorescens, Pseudomonas chlororaphis, Pseudomonas koreensis* and *Pseudomonas syringae* species, examples of culture media conventionally used for their culture include Tryptic Soy Agar (TSA, containing about 15 g/L agar, about 15 g/L pancreatic digest of casein, about 5 g/L sodium chloride and about 5 g/L peptic digest of soybean), Tryptic Soy Broth (TSB, containing about 17 g/L pancreatic digest of casein, about 5 g/L sodium chloride and about 3 g/L peptic digest of soybean, about 2.5 g/L dipotassium phosphate K₂HPO₄ and about 2.5 g/L of glucose), Lennox Broth (LB Lennox, containing about 10 g/L peptone, about 5 g/L yeast extract and about 5 g/L sodium chloride), Miller's Luria Bertani broth (Miller's LB broth, containing about 10 g/L peptone, about 5 g/L yeast extract and about 10 g/L sodium chloride), modified Miller's LB broth (modified Miller's LB broth, containing about 10 g/L peptone, about 5 g/L yeast extract and about 0.5 g/L sodium chloride), Mueller Hinton Broth (MHB, containing about 2 g/L beef infusion solids, about 1.5 g/L starch and about 17.5 g/L casein hydrolysate).

Appropriate conditions of culture may vary depending on the cell used, and a skilled person will know how to adapt the conditions depending on the selected cell. When using a *Pseudomonas* bacteria selected from the *Pseudomonas fluorescens, Pseudomonas chlororaphis, Pseudomonas koreensis* and *Pseudomonas syringae* species, conditions that will usually be appropriate include:
- an appropriate temperature (which will depend on the specific cell, for *Pseudomonas* strains from the *Pseudomonas fluorescens, Pseudomonas chlororaphis, Pseudomonas koreensis* and *Pseudomonas syringae* species, a suitable temperature is generally between 26°C and 30°C, such as about 28°C);
- when using a liquid culture, some shaking (such as about 100 to 220 rpm) may be used, in particular during the exponential phase of cellular growth, before reaching the stationary phase.

While non mandatory, conditions stimulating tailocin production by the cells may be used, in particular when the cell is a natural *Pseudomonas* bacterium selected from the *Pseudomonas fluorescens, Pseudomonas chlororaphis, Pseudomonas koreensis* and *Pseudomonas syringae* species that has not been engineered to increase tailocin production (for instance by replacing the native cluster promoter by a stronger promoter).

Tailocin production may notably be stimulated by any appropriate method, in particular by any appropriate method imposing a stress on the cultured cells, such as ultraviolet irradiation, anaerobic stress, heat induction or addition to the culture medium of a DNA damaging agent (such as mitomycin C, ciprofloxacin or H₂O₂).

Suitable ultraviolet irradiation conditions include exposition of cultured cells to UV using a UV lamp (e.g. 45 J/m2 at 10 cm distance for 10 min, or 15 W UV lamp at 40 cm distance for 5 seconds) or using a transilluminator (8 W UV-B light tubes for 4 min, or 360 µw cm⁻² s⁻¹ for 7 s).

Suitable anaerobic stress conditions include an oxygen-reduced or an oxygen-free environment generated by a transient incubation of cultured cells in an anaerobic incubator or with addition of paraffin in surface of the liquid culture.

Suitable heat induction conditions include non-lethal temperature shifts on cultured cells, such as, for *Pseudomonas* strains from the *Pseudomonas fluorescens, Pseudomonas chlororaphis, Pseudomonas koreensis* and *Pseudomonas syringae* species, shifting the culture temperature (generally between 26°C and 30°C, such as about 28°C) to a temperature of 32°C to 35°C.

When adding a DNA damaging agent to the culture medium, suitable concentrations ranges may be as follows:
- mitomycin C: 0.2 to 5 µg/mL, preferably 0.75 to 3 µg/mL, 1 to 2 µg/mL, such as about 1.5 µg/mL,
- ciprofloxacin: 10 to 0.01 µg/mL, preferably 5 to 0.1 µg/mL, 3 to 0.5 µg/mL, such as about 2 µg/mL,
- H₂O₂: 0.1 to 5 µg/mL, preferably 0.5 to 3 µg/mL, such as about 1 µg/mL.

When using a liquid culture, shaking may be stopped during the induction phase.

### Step (b)

In step (b), a raw cell lysate is obtained. The raw cell lysate may be obtained by natural or non-natural lysis.

In an embodiment, the raw cell lysate may be obtained by continuing the cell culture of step (a) until natural lysis of the cells occurs (natural lysis). Indeed, tailocin-producing cells will generally naturally lyse when cultured during a sufficient time. In this embodiment, natural lysis may be total or only partial, as total lysis is not necessary in order to obtain a cell lysate with antibacterial activity against *E. coli.* However, the yield in tailocin will be higher if higher cell lysis has occurred. Therefore, the culture duration may be optimized for optimal tailocin yield, by measuring the tailocin bactericidal activity of the lysate obtained at various culture times.

In another embodiment, the raw cell lysate may be obtained by lysing the cell culture of step (a). Such lysis may be performed mechanically using bead-beating, French's press, sonication, or chemically by enzymatic digestion. Here also, the lysis conditions may be optimized by measuring the tailocin bactericidal activity of the lysate obtained in various conditions.

Both methods may also be combined by continuing the cell culture of step (a) until at least some natural lysis of the cells occurs and further lysing a proportion of cells that have not yet naturally lysed.

### Step (c)

In step (c), the raw cell lysate of step (b) is filtered through a first filter with a pore size between 0,1 and 0,45 µm and the filtrate is collected.

The first filter has a pore size between 0,1 and 0,45 µm, preferably between 0.15 and 0.3 µm, and more preferably of about 0.2 µm, which permits to sterilize the cell lysate and to provide a first purification by removing big cell membrane debris.

Examples of appropriate first filters for step (c) include 0.2 µm cellulose acetate filters, 0.2 µm polyéthersulfone (PES) filters, 0.2 µm nitrocellulose filters, 0.2 µm polypropylene filters, and 0.2 µm polytétrafluoroéthylène filters.

### Step (d)

In step (d), the filtrate of step (c) is further filtered through a second filter with a cut-off between 50 and 300 kDa and the retentate is collected, thereby obtaining a purified cell lysate enriched in tailocin.

The second filter has a cut-off between 50 and 300 kDa, preferably between 75 and 200 kDa, between 75 and 150 kDa, such as about 100 kDa, which permits to enrich the retentate in tailocin(s), as most proteins have a molecular weight lower than the indicated cut-offs and will thus go through the filter and be no more present (or at very low concentration) in the retentate.

The purified lysate obtained at the end of step (d) is thus enriched in high molecular weight protein complexes and thus in tailocin(s).

### Preferred method

In a preferred embodiment, the purified cell lysate enriched in tailocin(s) has been obtained by a method comprising at least the steps of:
(a) cultivating the cell in a cell culture under conditions stimulating tailocin production by the cell selected from ultraviolet irradiation, anaerobic stress, heat induction or addition of a DNA damaging agent such as mitomycin C, ciprofloxacin, or H₂O₂ (preferably addition of mitomycin C) to the culture medium,
(b) obtaining a raw cell lysate by continuing the bacterial culture of step (a) until natural lysis of the cells,
(c) filtering the raw cell lysate of step (b) through a first filter with a pore size between 0,15 and 0,30 µm (preferably about 0.2 µm) and collecting the filtrate, and
(d) filtering the filtrate of step (c) through a second filter with a cut-off between 75 and 150 kDa (preferably about 100 kDa) and collecting the retentate, thereby obtaining a purified cell lysate enriched in tailocin.

### Preferred concentrations and activity of the composition

Preferably, the composition for use according to the invention comprises a purified cell lysate and as a result comprises a protein concentration (total protein concentration of all proteins present in the composition) of at least 1 ng.mL⁻¹, preferably at least 2 ng.mL⁻¹, at least 3 ng.mL⁻¹, at least 4 ng.mL⁻¹, at least 5 ng.mL⁻¹, at least 6 ng.mL⁻¹, at least 7 ng.mL⁻¹, at least 8 ng.mL⁻¹, at least 9 ng.mL⁻¹, at least 10 ng.mL⁻¹. However, a protein concentration higher than 10 mg.mL⁻¹ will generally not be useful to obtain antibacterial activity, and the composition for use according to the invention thus preferably comprises a protein concentration of at most 10 mg.mL⁻¹, preferably at most 5 mg.mL⁻¹, or at most 1 mg.mL⁻¹. The composition for use according to the invention thus preferably comprises a protein concentration between 1 ng.mL⁻¹ and 10 mg.mL⁻¹, preferably between 2 ng.mL⁻¹ and 5 mg.mL⁻¹, or between 3 ng.mL⁻¹ and 1 mg.mL⁻¹.

Alternatively or in combination with the total protein concentration, and optionally the proportion of tailocin(s) among total proteins, the composition for use according to the invention preferably comprises a significant bactericidal activity against at least one *E*. *coli* strain, and thus preferably shows a bactericidal activity on at least one *E. coli* strain at a concentration of at least 10 mg.mL⁻¹, preferably at a concentration of at least 1 mg/mL ng.mL⁻¹, at a concentration of at least 100 ng.mL⁻¹, or at a concentration of at least 10 ng.mL⁻¹, or at a concentration of at least 1 ng.mL⁻¹, wherein the concentration is expressed as the amount of total proteins per mL.

### Other components of the composition

The composition for use according to the invention is preferably a pharmaceutical composition and may thus contain various pharmaceutically acceptable excipient, notably intended to stabilize the tailocin(s) present in the composition. Pharmaceutically acceptable excipients intended to confer the desired consistency, depending on the drug format and its intended administration route, may also be present.

### Compositions comprising several lysates

The composition for use according to the invention comprises one or more cell lysates as described above.

A single tailocin will generally not show bactericidal activity against all *E. coli* strains, as its specificity is dependent on the interaction of its baseplate and associated receptor binding protein (RBP) with surface receptors of the target *E. coli* strain. As a result, in a preferred embodiment, the composition for use according to the invention may comprise at least 2, at least 3, at least 4, at least 5, at least 6, at least 7, at least 8, at least 9, or at least 10 (such as 2 to 100, 2 to 50, 2 to 40, 2 to 30, 2 to 25, 2 to 20, 3 to 20, 4 to 20, 5 to 20, 5 to 15) distinct cell lysates, wherein each distinct cell produces at least one tailocin from a *Pseudomonas* bacterium selected from the group consisting of *Pseudomonas fluorescens, Pseudomonas chlororaphis, Pseudomonas koreensis* and *Pseudomonas syringae* species.

In particular, the composition for use according to the invention may comprise at least 2, at least 3, at least 4, at least 5, at least 6, at least 7, at least 8, at least 9, at least 10, at least 11, at least 12, at least 13, at least 14 of or even the 15 bacterial lysates obtained from the *Pseudomonas fluorescens, Pseudomonas chlororaphis, Pseudomonas koreensis* and *Pseudomonas syringae* species described in **Table 1.**

Combinations of cell lysates showing complementary specificities against distinct *E. coli* species are particularly preferred. In addition, when two cell lysates have similar specificities, the cell lysate with bactericidal activity at a lower concentration is preferably selected. Although non limiting, the following combinations are of particular interest:
i) A cell lysate from *Pseudomonas fluorescens* strain Ps36, a cell lysate from *Pseudomonas chlororaphis subsp. piscium* strain Ps54, a cell lysate from *Pseudomonas chlororaphis* strain Ps6, a cell lysate from *Pseudomonas koreensis* strain Ps4 and a cell lysate from *Pseudomonas chlororaphis subsp. aureofaciens* strain Ps53, as a "species-specific" combination that should show bactericidal activity against all tested enteropathogenic and uropathogenic *E. coli* strains (see **Table 4** and **Table 5** in Example 1);
ii) A cell lysate from *Pseudomonas koreensis* strain Ps4, a cell lysate from *Pseudomonas chlororaphis subsp. aureofaciens* strain Ps53 and a cell lysate from *Pseudomonas chlororaphis subsp. chlororaphis* strain Ps38, as this combination should show bactericidal activity against all tested antibiotic-resistant *E. coli,* including the multi-drug resistant and highly virulent ST131 strain (i.e. EC118) (see **Table 4** and **Table 5** in Example 1);
iii) A cell lysate from *Pseudomonas fluorescens* strain Ps36, a cell lysate from *Pseudomonas chlororaphis* strain Ps6, a cell lysate from *Pseudomonas koreensis* strain Ps4 and a cell lysate from *Pseudomonas chlororaphis subsp. aureofaciens* strain Ps53, and a cell lysate from *Pseudomonas fluorescens* strain Ps40 as an "anti-UPEC" combination that should show bactericidal activity against all tested uropathogenic *E. coli* strains belonging to B2 and D phylogroups (see **Table 4** and **Table 5** in Example 1);
iv) A cell lysate from *Pseudomonas fluorescens* strain Ps36 and cell lysate from *Pseudomonas chlororaphis* strain Ps6, as an "anti-O157:H7" combination that should show bactericidal activity against all tested enteropathogenic *E. coli* serotype O157:H7 (see **Table 4** and **Table 5** in Example 1).

Combinations i) is particularly useful for the prevention or treatment (preferably the prevention) of gastrointestinal contaminations or infections by *E. coli* strains and may thus notably be included in food supplements. They are also particularly useful for the prevention or treatment (preferably the prevention) of urogenital contaminations or infections by *E. coli* strains and may thus notably be included in an intimate care composition.

Combination ii) is particularly useful for the prevention or treatment (preferably the prevention) of urogenital contaminations or infections by antibiotic-resistant *E. coli* strains and may thus notably be included in an intimate care composition.

Combination iii) is particularly useful for the prevention or treatment (preferably the prevention) of urogenital contaminations or infections by uropathogenic *E. coli* strains belonging to the most prevalent phylogroups (i.e. B2 and D) and may thus notably be included in an intimate care composition.

Combination iv) is particularly useful for the prevention or treatment (preferably the prevention) of gastrointestinal contaminations or infections by *E. coli* strains serotype O157:H7 and may thus notably be included in food supplements.

In addition to one or more (and up to all 15) bacterial lysates obtained from the *Pseudomonas fluorescens, Pseudomonas chlororaphis, Pseudomonas koreensis* and *Pseudomonas syringae* species described in **Table 1,** the composition for use according to the invention may further comprise bacterial lysates obtained from the *Pseudomonas fluorescens, Pseudomonas chlororaphis, Pseudomonas koreensis* and *Pseudomonas syringae* species described in **Table 2** or from any other bacteria from the *Pseudomonas fluorescens, Pseudomonas chlororaphis, Pseudomonas koreensis* and *Pseudomonas syringae* species producing one or more tailocins, or even from any other bacteria from other species producing one or more tailocins.

The other bacterial lysates will preferably be selected from bactericidal activity against at least one *E. coli* strain.

### Contamination or infection by E. coli

The composition for use according to the invention is for use in the prevention or the treatment in a subject of a contamination or an infection by an *Escherichia coli* strain.

The composition for use according to the invention may be used for the prevention or the treatment in a subject of a contamination or an infection by any *Escherichia coli* strain. However, it will preferably be used in the prevention or the treatment in a subject of a contamination or an infection by an *Escherichia coli* strain known to be particularly associated with urogenital or gastrointestinal infections. Therefore, the composition for use according to the invention may preferably be used for the prevention or the treatment in a subject of a contamination or an infection by an *Escherichia coli* strain that:
(a) has a serotype selected from the group consisting of O157:H7, O44:H18, O111:K58(B4) and O26:H11;
(b) belongs to phylogroups B2 or D;
(c) belongs to the sequence type ST131; or
(d) any combination of (a) to (c).

*E. coli* strains (a) with a serotype selected from the group consisting of O157:H7, O44:H18, O111:K58(B4) and O26:H11 are known to be particularly associated with gastrointestinal infections or the enteroaggregative phenotype (Alharbi et al., 2022, Bumunang et al., 2023), while *E. coli* strains (b) belonging to phylogroups B2 or D (Kot, 2019) or (c) belonging to the sequence type ST131 (Lavigne et al., 2012, Nicolas-Chanoine et al., 2014) are known to be particularly associated with urogenital infections.

Regarding the type of contamination or infection, the composition for use according to the invention may preferably be used in the prevention in a subject of a urogenital infection or gastrointestinal infection.

When intended for the prevention in a subject of a urogenital infection, the composition will preferably be formulated as an intimate care product.

When intended for the prevention in a subject of a gastrointestinal infection, the composition will preferably be formulated as a food supplement.

### Non-therapeutic uses of a Pseudomonas fluorescens, Pseudomonas chlororaphis, Pseudomonas koreensis or Pseudomonas syringae lysate comprising at least one tailocin against contamination by an Escherichia coli strain

The present invention also relates to the use of a composition comprising a cell lysate, wherein the cell produces at least one tailocin from a *Pseudomonas* bacterium selected from the group consisting of *Pseudomonas fluorescens, Pseudomonas chlororaphis, Pseudomonas koreensis* and *Pseudomonas syringae* species, for the prevention and/or the treatment of a contamination by an *Escherichia coli* strain in a food product or on a surface. Such uses are non-therapeutic uses.

### Prevention and/or the treatment of a contamination by an Escherichia coli strain in a food product

In this non-therapeutic use, a food product is treated with the composition in order to kill *E. coli* bacteria that may be present in the food product.

Food product that may be contaminated by *E. coli* bacteria are not particularly limited and the treatment may thus be applied to any type of food product, be it a raw food product (meat, fish, shellfish, vegetables, fruits, cereals...) or a transformed food product (pizza, ready to use meals...). However, minced beef, uncooked meat (in particular uncooked beef and uncooked lamb) or unpasteurized dairy products (in particular raw milk and raw-milk cheese), raw vegetables and salads (Erickson and Doyle, 2007) are known to be particularly susceptible to contamination by *E. coli* bacteria and the composition may thus particularly be used for prevention and/or the treatment of a contamination by an *Escherichia coli* strain in these food products.

The composition may be used contacted with the food product during food processing, before packaging or just before use.

The composition may notably be used before packaging of ready to cook raw or marinated food products.

### Prevention and/or the treatment of a contamination by an Escherichia coli strain on a surface

In this non-therapeutic use, a surface is treated with the composition in order to kill *E. coli* bacteria that may be present on the surface.

Surfaces that may be treated are not particularly limited and notably include both inorganic (e.g. metals, minerals...) and organic (e.g. plants, leather, wool, coton, fur...) surfaces, as well as surfaces from natural (e.g. metal items, mineral items, leather items, wool items, cotton items, fur items ...) or non-natural synthetic (e.g. plastics, synthetic fabrics, composite materials...) materials. The only excluded surfaces are living body parts of an animal or human subject (e.g. living skin is excluded, but leather, which is obtained from skin but is a non-living material, is not excluded) as this use is non-therapeutic.

### Cell and tailocin

Any embodiment disclosed in the sub-section "Cell and tailocin" of the above section relating to a composition for use according to the invention may be applied similarly to the non-therapeutic uses according to the invention.

### Cell lysate

Any embodiment disclosed in the sub-section "Cell lysate" of the above section relating to a composition for use according to the invention may be applied similarly to the non-therapeutic uses according to the invention.

### Preferred concentrations and activity of the composition

Any embodiment disclosed in the sub-section "Preferred concentrations and activity of the composition" of the above section relating to a composition for use according to the invention may be applied similarly to the non-therapeutic uses according to the invention.

### Compositions comprising several lysates

Any embodiment disclosed in the sub-section "Compositions comprising several lysates" of the above section relating to a composition for use according to the invention may be applied similarly to the non-therapeutic uses according to the invention.

### Contamination or infection by E. coli

Any embodiment disclosed in the sub-section "Contamination or infection by E. coli" of the above section relating to a composition for use according to the invention may be applied similarly to the non-therapeutic uses according to the invention.

When the use is for the prevention and/or the treatment of a contamination by an *Escherichia coli* strain in a food product, cell lysates from cells production tailocin(s) with bactericidal activity against *E. coli* strains known to be particularly associated with gastrointestinal infections (such as *E. coli* serotypes O157:H7, O44:H18, O111:K58(B4) and O26:H11) may preferably be comprised in the composition.

### Intimate care product

The present invention also relates to an intimate care product comprising one or more cell lysate(s) as defined herein.

Such an intimate care product will be useful for and may thus be for use in the prevention of urogenital contaminations and infections by *E. coli* strains.

### Cell and tailocin

Any embodiment disclosed in the sub-section "Cell and tailocin" of the above section relating to a composition for use according to the invention may be applied similarly to the intimate care product according to the invention.

As the intimate care product is preferably intended for the prevention of urogenital contaminations and infections by *E. coli* strains, it will preferably comprise one or more cell lysates from cells producing tailocin(s) with bactericidal activity against *E. coli* strains known to be particularly associated with urogenital contaminations and infections by *E. coli* strains, in particular against at least one *E. coli* strain belonging to phylogroups B2 or D (Kot, 2019) or to the sequence type ST131 (Lavigne et al., 2012, Nicolas-Chanoine et al., 2014).

### Cell lysate

Any embodiment disclosed in the sub-section "Cell lysate" of the above section relating to a composition for use according to the invention may be applied similarly to the intimate care product according to the invention.

### Preferred concentrations and activity of the composition

Any embodiment disclosed in the sub-section "Preferred concentrations and activity of the composition" of the above section relating to a composition for use according to the invention may be applied similarly to the intimate care product according to the invention.

### Compositions comprising several lysates

Any embodiment disclosed in the sub-section "Compositions comprising several lysates" of the above section relating to a composition for use according to the invention may be applied similarly to the intimate care product according to the invention.

### Food supplement

The present invention also relates to a food supplement comprising one or more cell lysate as defined herein.

Such a food supplement will be useful for and may thus be for use in the prevention of gastrointestinal contaminations and infections by *E. coli* strains.

### Cell and tailocin

Any embodiment disclosed in the sub-section "Cell and tailocin" of the above section relating to a composition for use according to the invention may be applied similarly to the food supplement according to the invention.

As the food supplement is preferably intended for the prevention of gastrointestinal contaminations and infections by *E. coli* strains, it will preferably comprise one or more cell lysates from cells producing tailocin(s) with bactericidal activity against *E. coli* strains known to be particularly associated with gastrointestinal contaminations and infections by *E. coli* strains, in particular against at least one *E. coli* strain of the O157:H7, O44:H18, O111:K58(B4) or O26:H11 serotype.

### Cell lysate

Any embodiment disclosed in the sub-section "Cell lysate" of the above section relating to a composition for use according to the invention may be applied similarly to the food supplement according to the invention.

### Preferred concentrations and activity of the composition

Any embodiment disclosed in the sub-section "Preferred concentrations and activity of the composition" of the above section relating to a composition for use according to the invention may be applied similarly to the food supplement according to the invention.

### Compositions comprising several lysates

Any embodiment disclosed in the sub-section "Compositions comprising several lysates" of the above section relating to a composition for use according to the invention may be applied similarly to the food supplement according to the invention.

### New tailocin cluster from Pseudomonas fluorescens Ps36, derivative products and uses thereof

### New tailocin cluster from Pseudomonas fluorescens Ps36

Among the tailocins produced by *Pseudomonas fluorescens, Pseudomonas chlororaphis, Pseudomonas koreensis* and *Pseudomonas syringae* species, the inventors have identified that *Pseudomonas fluorescens* Ps36 strains produces two original tailocins with relatively low sequence identity with known tailocins (see Example 1).

The present invention thus also relates to an isolated nucleic acid molecule comprising, consisting essentially of or consisting of a nucleotide sequence with:
(a) at least 80%, preferably at least 85%, more preferably at least 90%, at least 91%, at least 92%, at least 93%, at least 94%, at least 95%, at least 96%, at least 97%, at least 98%, at least 99%, at least 99.5% or even 100% identity with SEQ ID NO:1,
(b) at least 80%, preferably at least 85%, more preferably at least 90%, at least 91%, at least 92%, at least 93%, at least 94%, at least 95%, at least 96%, at least 97%, at least 98%, at least 99%, at least 99.5% or even 100% identity with SEQ ID NO:2,
(c) at least 80%, preferably at least 85%, more preferably at least 90%, at least 91%, at least 92%, at least 93%, at least 94%, at least 95%, at least 96%, at least 97%, at least 98%, at least 99%, at least 99.5% or even 100% identity with SEQ ID NO:3, or
(d) at least 80%, preferably at least 85%, more preferably at least 90%, at least 91%, at least 92%, at least 93%, at least 94%, at least 95%, at least 96%, at least 97%, at least 98%, at least 99%, at least 99.5% or even 100% identity with SEQ ID NO:5.

SEQ ID NO:1 contains 17544 bp and corresponds to the part of the cluster comprising ORFs encoding the fluorescin type (TC1) tailocin.

SEQ ID NO:2 contains 17469 bp and corresponds to the part of the cluster comprising ORFs encoding the syringacin type (TC2) tailocin.

SEQ ID NO:3 contains 35013 bp and corresponds to the complete gene cluster identified between the *mutS* and *cinA* genes, with ORFs encoding the fluorescin type (TC1) and the syringacin type (TC2) tailocins. SEQ ID NO:3 thus comprises both SEQ ID NO:1 and SEQ ID NO:2. High percentage identity with SEQ ID NO:3 is preferred.

SEQ ID NO:4 contains 1064 bp and corresponds to the two lysis genes (endolysin and spanin) located in 3' of ORFs encoding the syringacin type (TC2) tailocin.

SEQ ID NO:5 contains 36077 bp and corresponds to the complete gene cluster identified between the *mutS* and *cinA* genes, with ORFs encoding the fluorescin type (TC1), the syringacin type (TC2) tailocins and the two lysis genes. SEQ ID NO:5 thus comprises SEQ ID NO:1, SEQ ID NO:2 and SEQ ID NO:4. High percentage identity with SEQ ID NO:4 is also preferred.

In addition to a high percentage identity with SEQ ID NO:1, SEQ ID NO:2, SEQ ID NO:3, or SEQ ID NO:5 (preferably SEQ ID NO:3 or SEQ ID NO:5), the isolated nucleic acid molecule according to the invention preferably encodes proteins with amino acid sequences having at least 80%, preferably at least 85%, more preferably at least 90%, at least 91%, at least 92%, at least 93%, at least 94%, at least 95%, at least 96%, at least 97%, at least 98%, at least 99%, at least 99.5% or even 100% identity with the amino acid sequences of corresponding proteins encoded by SEQ ID NO:1, SEQ ID NO:2, SEQ ID NO:3 or SEQ ID NO:5 (preferably SEQ ID NO:3 or SEQ ID NO:5), respectively.

The amino acid sequences of proteins encoded by SEQ ID NO:1 are SEQ ID NO:6 to 25.

The amino acid sequences of proteins encoded by SEQ ID NO:2 are SEQ ID NO:26 to 47.

The amino acid sequences of proteins encoded by SEQ ID NO:3 are SEQ ID NO:6 to 47.

The amino acid sequences of proteins encoded by SEQ ID NO:4 are SEQ ID NO:48 to 49.

The amino acid sequences of proteins encoded by SEQ ID NO:5 are SEQ ID NO:6 to 49.

In particular, the isolated nucleic acid molecule according to the invention preferably comprises, consists essentially of or consists of a nucleotide sequence having at least 80% identity with SEQ ID NO:1, SEQ ID NO:2, SEQ ID NO:3 or SEQ ID NO:5 (preferably SEQ ID NO:3 or SEQ ID NO:5) and encoding proteins with amino acid sequences having at least 80% identity with the amino acid sequences of corresponding proteins encoded by SEQ ID NO:1 (i.e. SEQ ID NO:6 to 25), SEQ ID NO:2 (i.e. SEQ ID NO:26 to 47), SEQ ID NO:3 (i.e. SEQ ID NO:6 to 47) or SEQ ID NO:5 (i.e. SEQ ID NO:6 to 49) (preferably SEQ ID NO:3 or SEQ ID NO:5), respectively. More preferably, the isolated nucleic acid molecule according to the invention comprises, consists essentially of or consists of a nucleotide sequence having at least 90% identity with SEQ ID NO:1, SEQ ID NO:2, SEQ ID NO:3 or SEQ ID NO:5 (preferably SEQ ID NO:3 or SEQ ID NO:5) and encoding proteins with amino acid sequences having at least 90% identity with the amino acid sequences of corresponding proteins encoded by SEQ ID NO:1, SEQ ID NO:2, SEQ ID NO:3 or SEQ ID NO:5 (preferably SEQ ID NO:3 or SEQ ID NO:5), respectively. Even more preferably, the isolated nucleic acid molecule according to the invention comprises, consists essentially of or consists of a nucleotide sequence having at least 95% identity with SEQ ID NO:1, SEQ ID NO:2, SEQ ID NO:3 or SEQ ID NO:5 (preferably SEQ ID NO:3 or SEQ ID NO:5) and encoding proteins with amino acid sequences having at least 95% identity with the amino acid sequences of corresponding proteins encoded by SEQ ID NO:1, SEQ ID NO:2, SEQ ID NO:3 or SEQ ID NO:5 (preferably SEQ ID NO:3 or SEQ ID NO:5), respectively.

### Expression vector

The present invention also relates to an expression vector comprising the nucleic acid molecule according to the invention.

Any expression vector appropriate for expression of the fluorescin type (TC1) tailocin encoded by SEQ ID NO:1, the syringacin type (TC2) tailocin encoded by SEQ ID NO:2, both the fluorescin type (TC1) tailocin and the syringacin type (TC2) tailocin encoded by SEQ ID NO:3, or the complete cluster encoded by SEQ ID NO:5 may be used.

In view of the sizes of SEQ ID NO:1, SEQ ID NO:2 and even more SEQ ID NO:3 and SEQ ID NO:5, particularly preferred expression vectors are selected from episomal vectors and BACs. The expression vector according to the invention may notably be selected from any type of episomal vector or BAC described in the sub-section "Cell and tailocin" of the above section relating to a composition for use according to the invention.

### Cell

The present invention also relates to a cell comprising a nucleic acid molecule or an expression vector according to the invention.

In an embodiment, the cell according to the invention is *Pseudomonas fluorescens* strain Ps36 deposited at Collection nationale de cultures de microorganismes (CNCM, Institut Pasteur, 25-28 rue du Docteur Roux, 75724 Paris Cedex 15, France) on the 21^{st} of March 2024 under accession number I-6068.

Alternatively, the cell according to the invention is a recombinant cell (any type of recombinant cell described above in the sub-section "Cell and tailocin" of the above section relating to a composition for use according to the invention), preferably a recombinant bacterium (any type of recombinant bacterium described above in the sub-section "Cell and tailocin" of the above section relating to a composition for use according to the invention), transformed by a nucleic acid molecule according to the invention, or an expression vector according to the invention.

### Cell lysate and method for preparing it

The present invention also relates to a cell lysate comprising at least one tailocin obtained from the cell according to the invention.

The present invention also relates to a method for preparing a cell lysate enriched in tailocin comprising at least the steps of:
(a) cultivating the cell according to the invention, preferably under conditions stimulating tailocin production by the bacteria, preferably tailocin production is stimulated by ultraviolet irradiation, anaerobic stress, heat induction or addition of a DNA damaging agent such as mitomycin C, ciprofloxacin, or H₂O₂ to the culture medium,
(b) continuing the culture until natural lysis of the cell of step a) or lysing the cell of step a) to obtain a cell lysate,
(c) filtering the cell lysate of step (b) through a filter with a pore size between 0,1 and 0,45 µM and collecting the filtrate, and
(d) filtering the filtrate of step (c) through a filter with a cut-off between 50 and 300 kDa and collecting the retentate, thereby obtaining a cell lysate enriched in tailocin.

For each of the steps (a) to (d), any embodiment disclosed in the sub-section "Cell lysate" of the above section relating to a composition for use according to the invention may be used.

The present invention also relates to a cell lysate obtained by the method for preparing a cell lysate enriched in tailocin according to the invention.

### (Pharmaceutical) composition

The present invention also relates to a composition comprising a cell lysate according to the invention. The composition is preferably a pharmaceutical composition, and any pharmaceutically acceptable excipient, notably intended to stabilize the tailocin(s) or to confer the desired consistency, depending on the drug format and its intended administration route, may also be present.

### Therapeutic uses of the cell lysate

The present invention also relates to a cell lysate according to the invention, for use in the prevention or the treatment in a subject of a contamination or an infection by an *Escherichia coli* strain, preferably selected from the group consisting of urogenital infection or gastrointestinal infection.

The present invention also relates to the use of a cell lysate according to the invention for manufacturing a drug for use in the prevention or the treatment in a subject of a contamination or an infection by an *Escherichia coli* strain, preferably selected from the group consisting of urogenital infection or gastrointestinal infection.

The present invention also relates to the use of a cell lysate according to the invention for the prevention or the treatment in a subject of a contamination or an infection by an *Escherichia coli* strain, preferably selected from the group consisting of urogenital infection or gastrointestinal infection.

The present invention also relates to a method of treating or preventing a contamination or an infection by an *Escherichia coli* strain in a subject in need thereof, comprising administering to the subject a therapeutically efficient amount of a cell lysate according to the invention.

Any preferred embodiment disclosed in the sub-section "Contamination or infection by E. coli" of the above section relating to a composition for use according to the invention is also preferred for the cell lysate or composition according to the invention.

### Non-therapeutic uses of the cell lysate

The present invention also relates to the use of a cell lysate according to the invention or of a composition comprising it for the prevention or the treatment of a contamination by an *Escherichia coli* strain in a food product or on a non-living surface.

Any embodiment disclosed in the sub-section "Prevention and/or the treatment of a contamination by an *Escherichia coli* strain in a food product" of the section above relating to the general non-therapeutic use according to the invention also applies to the non-therapeutic use of a cell lysate according to the invention or of a composition comprising it.

The following examples merely intend to illustrate the present invention.

### EXAMPLES

### Example 1: Tailocins produced by various Pseudomonas fluorescens, Pseudomonas chlororaphis, Pseudomonas koreensis and Pseudomonas syringae species show bactericidal activity against E. coli strains

### Materials and Methods

### Bacterial strains and growth conditions

Strains used in this study are detailed in the **Table 3.**

*Pseudomonas* strains were routinely grown on Tryptic Soy Agar (TSA), or in Tryptic Soy Broth (TSB) at 28 °C. *E. coli* strains were routinely grown on LB Lennox Agar (LBA), or in Lennox broth (LB) at 37 °C. For double layer agar assays, LB was supplemented with 0.6% agar (LB soft agar; LBS). All liquid cultures were incubated with shaking at 150 rpm. Minimum Bactericidal Concentration (MBC) assays were performed in MHB growth medium.

All strains were stored at -80 °C in 25% glycerol [v/v].

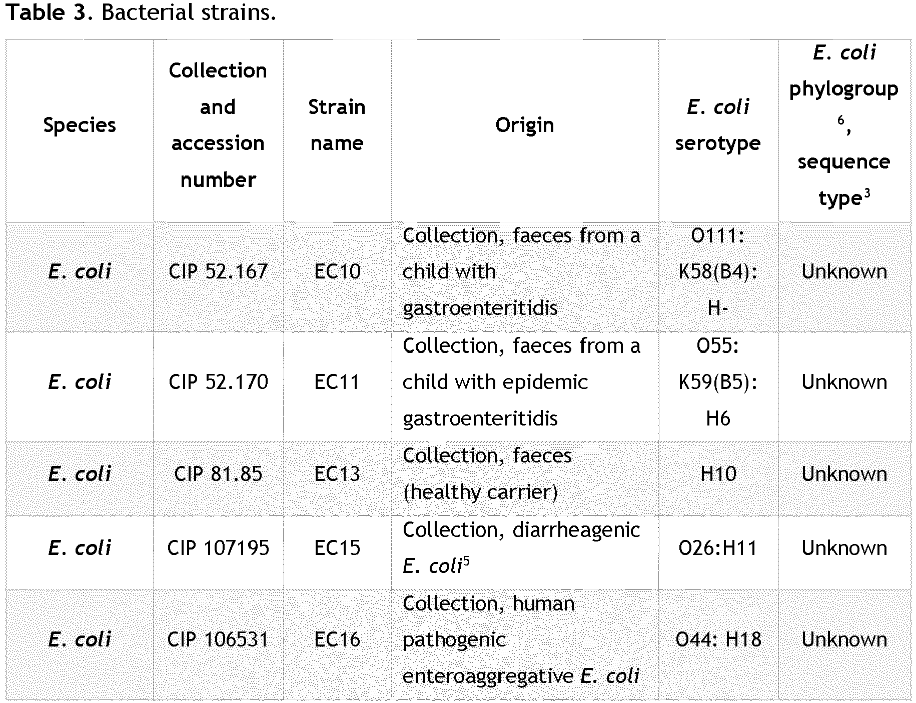

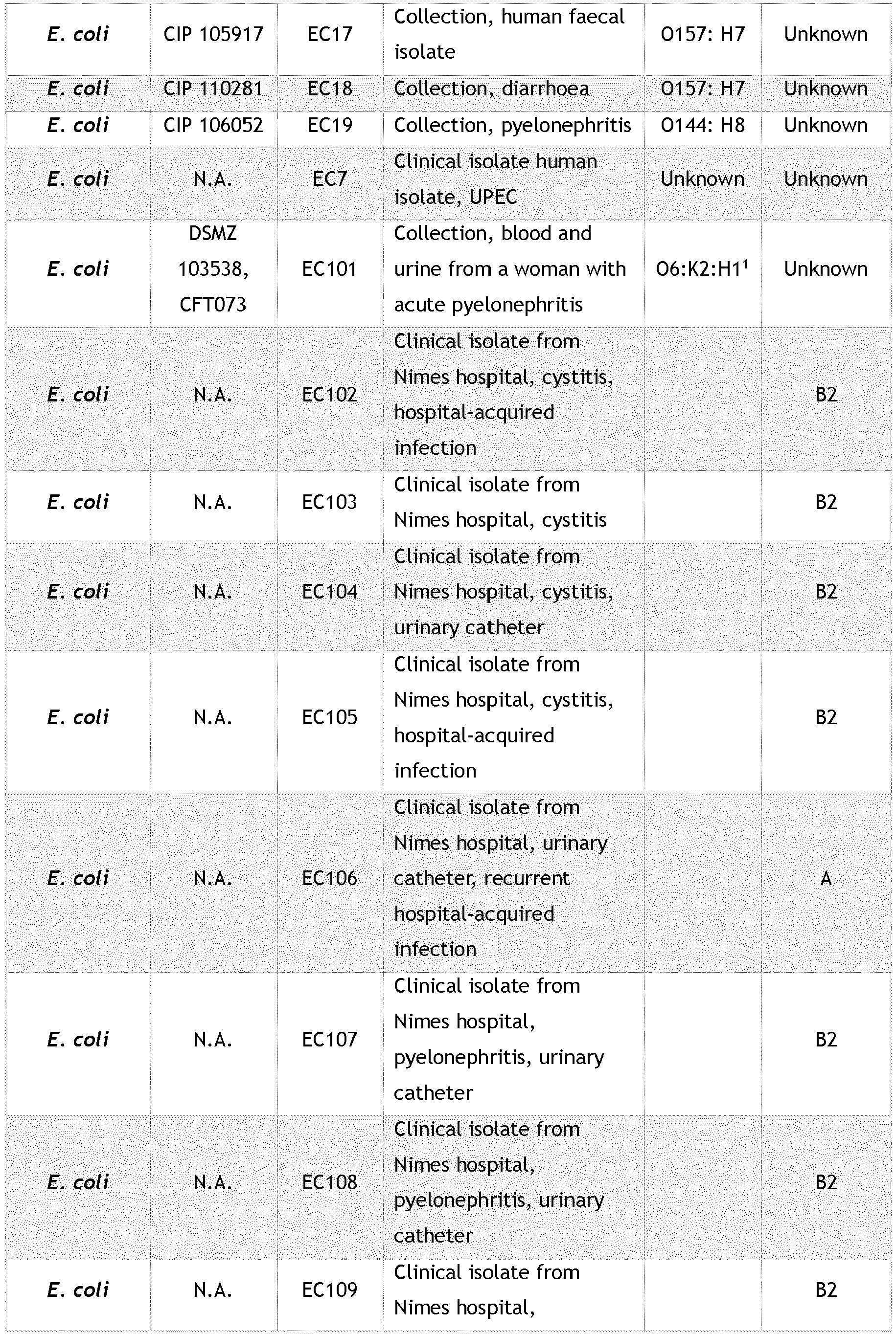

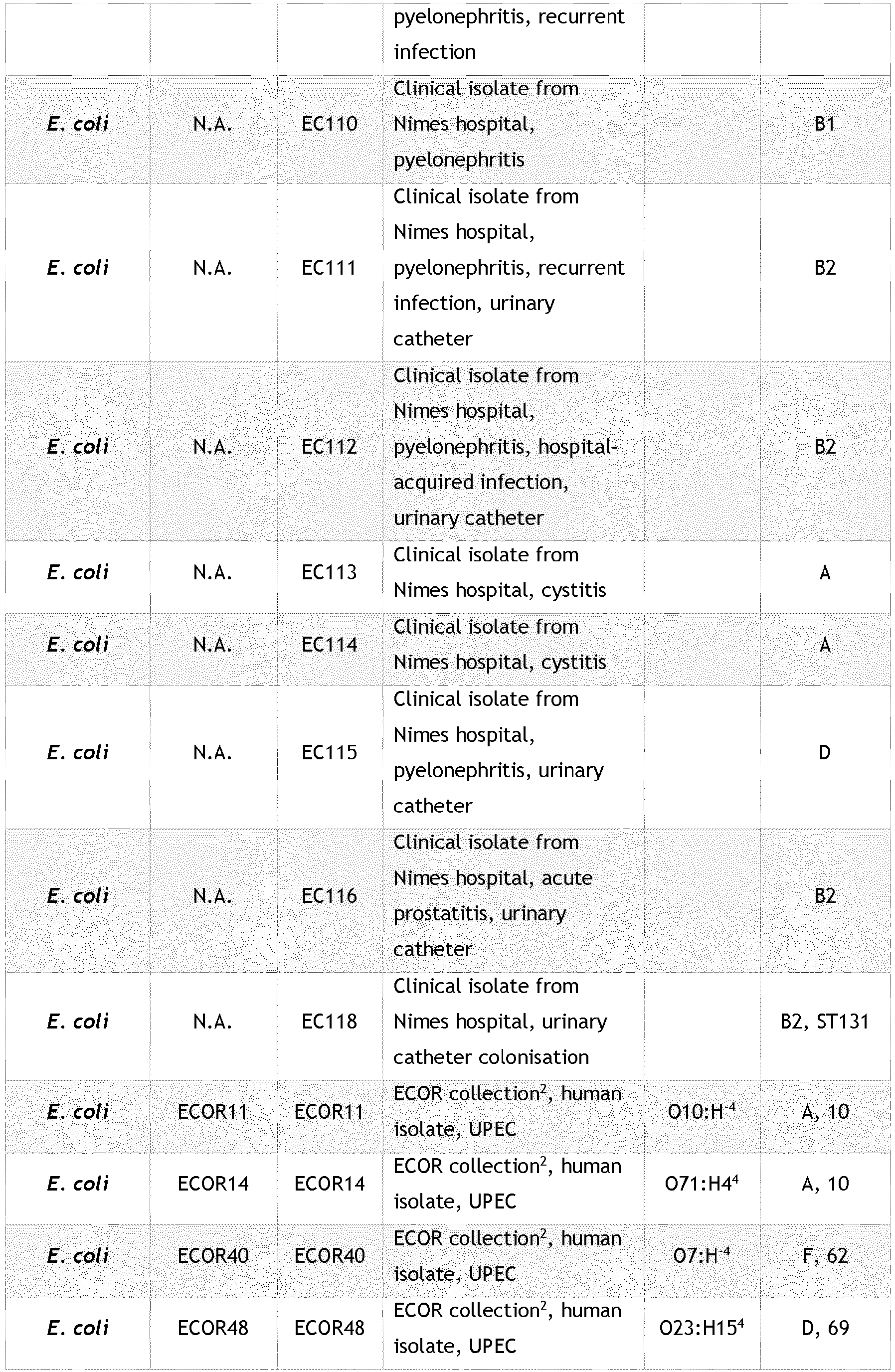

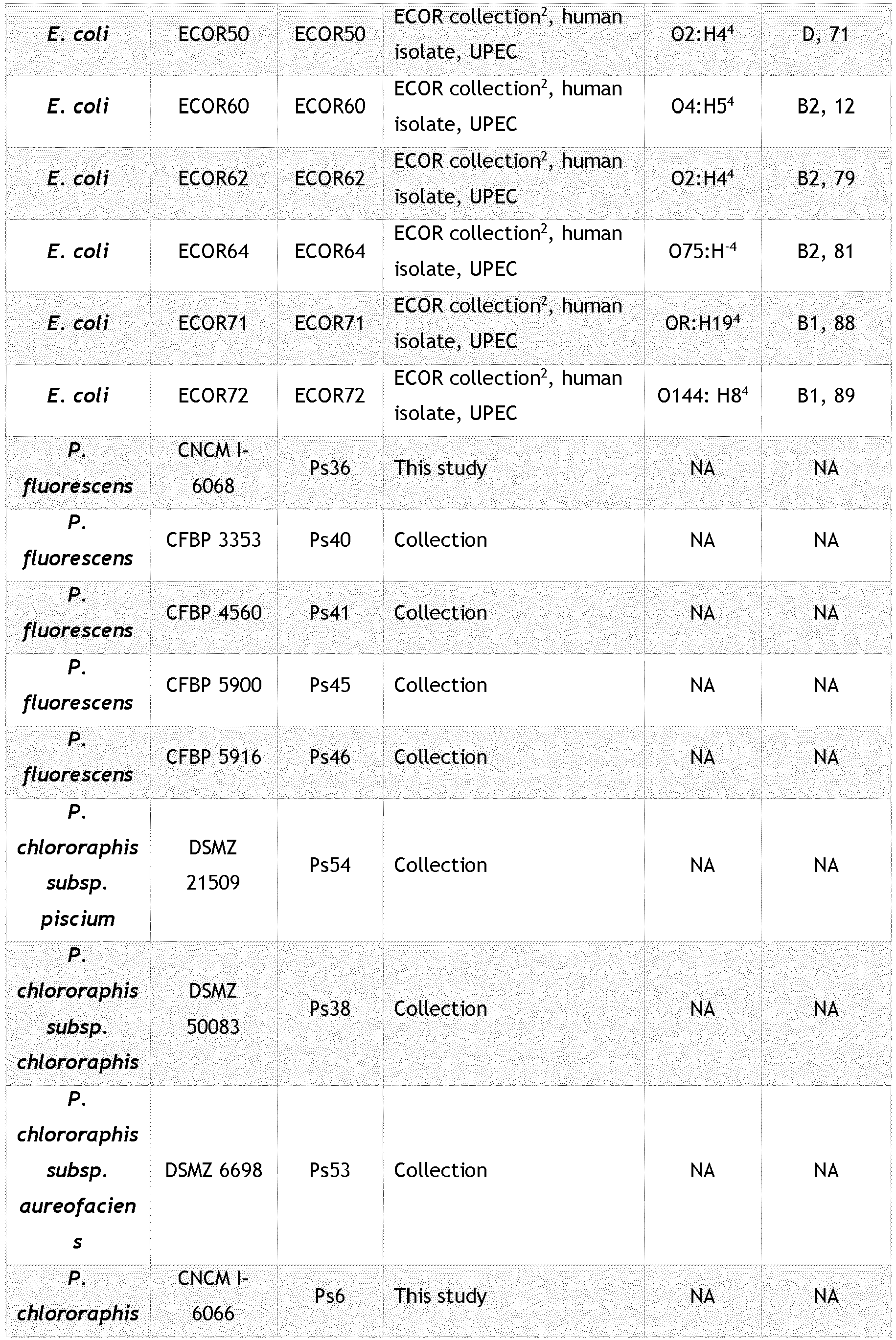

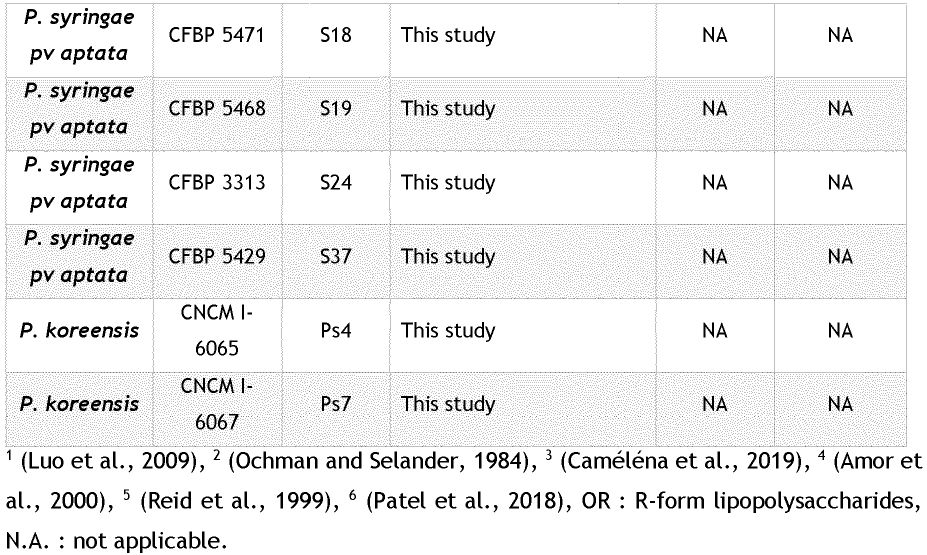

### Bacterial identification

The 16S rRNA gene of bacterial targets was amplified by PCR using 0.2 mM primers (27F: AGAGTTTGATCCTGGCTCAG (SEQ ID NO:50); 1492R: GGTTACCTTGTTACGACTT (SEQ ID NO:51)), 400 mM of each dNTP, 3 % DMSO, 1 X Xtreme buffer, 1 U KOD hot start polymerase, and 4 µL of bacterial lysate. Amplification conditions were as follows: 5 min at 95 °C to denature the DNA, followed by 30 cycles of denaturation at 95 °C for 30 sec, primer annealing at 58 °C for 30 sec and strand extension at 72 °C for 1min 30 sec, and a final extension at 72°C for 5 min on a VWR XT-96 thermal cycler. PCR products were separated on a 1 % agarose gel and DNA bands were visualized with ClearSight DNA stain (EUROMEDEX). The PCR products were then submitted to purification and DNA sequencing (Eurofins Genomics, Germany) using the primers 27F and 1492R. Sequencing reads were assembled using BioEdit (Alzohairy, 2011) to obtain one contig per strain. Molecular identification was achieved by comparing consensus sequences to a database library of known 16S rRNA gene sequences in GenBank (https://blast.ncbi.nlm.nih.gov/). Sequences with the highest maximum identity score and representing the majority species group among the 100 best hits determined the identification. 16S rRNA gene sequences were designated as: SEQ ID NO:52 for strain Ps36, SEQ ID NO:53 for strain Ps4, SEQ ID NO:54 for strain Ps7, and SEQ ID NO:55 for strain Ps6.

### Tailocins extraction and purification

To extract and purify tailocins from the different *Pseudomonas* strains, cultures were restarted into fresh TSB from an overnight culture. To induce the production of tailocins, 1.5 µg/mL of mitomycin C was added to the culture. The cultures were further incubated at 28 °C with shaking (150 rpm) for one night, and then centrifuged at 5000 g for 10 min at 4 °C. Supernatants were filtered with 0.2 µm cellulose acetate filters. Tailocin particles present in the supernatant were purified onto Vivaspin 20 Centrifugal Concentrators Polyethersulfone (Sartorius) with a molecular weight cut-off of 100 kDa. Particles were watched one time with 10 mL Tn50 buffer (50mM NaCl, 10mM Tris-HCl (pH=7)), and then resuspended from the filters by using 700 µL of Tn50 buffer. Amounts of purified proteins were quantified by using a Nanovue Plus spectrophotometer (GE HealthCare).

### Testing bacterial sensitivity to tailocin particles

To test the sensitivity of the numerous different bacterial strains, soft agar assays were performed. Following an overnight incubation, cultures were restarted into fresh LB until reach an OD₆₀₀ₙₘ of 0.8. Then, 900 µL of each bacterial culture was mixed with 30 mL of melted LBS. This mixture was poured into an empty Petri dish, and aliquots of 5 µL of purified particles suspension as well as serial dilutions of the purified particles were deposited once the agar solidified. The plates were incubated overnight at 37 °C. The sensitivity of the bacterial strains toward the purified particles was assessed using a scale ranging from clear lysis to opaque lysis versus no lysis (resistant bacteria). The experiment was performed independently twice and was read by two independent people.

The sensitivity of target strains towards tailocins was evaluated in liquid assays by evaluation of the minimum concentration of tailocins sufficient to kill bacterial targets (MBC). MBC assays were carried out using the broth microdilution method in accordance with the Clinical and Laboratory Standards Institute (CLSI) and the European Society of Clinical Microbiology and Infectious Diseases (EUCAST) guidelines with slight modifications. *E. coli* targets were restarted from an overnight culture in fresh MHB. At OD₆₀₀ₙₘ = 0.2, cultures in exponential phase were adjusted at theoretical concentrations of 1 to 5 × 10⁵ CFU/mL (CFU, colonies forming units). 50 µL of each bacterial suspension were mixed to 50 µL of double dilutions of tailocins in a microtiter plate. The last well of each line was left with no tailocin to be used as a growth control during testing. In parallel, 10 µL of diluted bacterial suspensions were spotted on LBA to determine the number of viable cells before treatment. Plates were incubated at 28 °C for 16-19 hours.

After incubation, 10 µL-spots were counted to validate the initial inoculum. In addition, aliquots of 10 µL of each microplate wells were mixed with 90 µL of fresh LB medium in a new microtiter plate. Plate was incubated at 28°C with linear shaking (5 Hz, amplitude 15 mm) in a microplate reader (Multiskan SkyHigh Spectrophotometer, ThermoFisher Scientific), and optical density at 600 nm was recorded for several hours. Minimum bactericidal concentration was defined as the lowest concentration of tailocins showing no bacterial survival in these conditions. Experiments were done in duplicate.

### Activity testing of a phage cocktail targeting antibiotic-resistant UPEC

Tailocin cocktail was designed based on our findings of tailocin combinations with demonstrated lytic efficacy against antibiotic-resistant UPEC. The purified tailocins produced by *P. chlororaphis* Ps38, *P. chlororaphis* Ps53 *and P. koreensis* Ps4 were diluted to obtain 5.35 mg/mL final concentration for each. Then, the three purified tailocins were mixed at ratio 1:1:1 to compose the cocktail. The sensitivity of the 8 antibiotic-resistant UPEC was evaluated in soft agar assays as described above. Experiment was performed in technical duplicate with the tailocin cocktail as well as individual tailocins.

### Results

### Tailocins isolated from P. fluorescens, P. chlororaphis, P. koreensis and P. syringae species have a strong specific bactericidal activity on E. coli

The antimicrobial activity of a wide panel of tailocins from *P. fluorescens, P. chlororaphis, P. koreensis* and *P. syringae* was screened semi-quantitatively by a dilution method. Serial dilutions of purified lysates of each of the tested *Pseudomonas* strains were spotted onto bacterial lawns of relevant *E. coli* strains. Two groups of *E. coli* were selected:
i) **Enteropathogenic *E. coli* and/or strains isolated from faeces.** This group notably includes strains of the EHEC group belonging to the serotype O157:H7 (i.e. EC17 and EC18), a major pathogenic foodborne serotype responsible for the Hamburger disease, as well as to the serotype O26:H11 (i.e. EC15), which has emerged as the most important non-O157:H7 EHEC, with respect to their ability to cause diarrhoea and the hemolytic uraemic syndrome (Bielaszewska et al., 2007). Strain EC16 (serotype O44:H18) was selected to represent pathogenic EAEC while EC10 (O111), EC11 (O55), and EC15 (O26) belong to EPEC. Finally, EC13 is an additional strain isolated from faeces from a healthy carrier.
ii) **Uropathogenic *E. coli* (UPEC).** This selection notably comprises 10 UPEC strains of the ECOR collection. This collection contains isolates designed to represent the variation and genetic structure of *E. coli* in natural populations. These 10 strains belong to the main four phylogroups of the species (i.e. A, B1, B2 and D). It has been reported that UPEC mainly belongs to phylogroups B2 and D, while phylogroups A and B1 are commensals (Picard et al., 1999; Munkhdelger et al., 2017). In addition, EC101 (CFT073) is an archetypal extraintestinal pathogenic *E. coli* (ExPEC) isolated from a patient with acute pyelonephritis. Clinical UPEC strains complete this selection. This panel of isolates represents a multiplicity of the infection sites and associated diseases (cystitis, pyelonephritis, etc) and compiled different levels of antibiotic resistance. It notably includes fluoroquinolones-resistant UPEC as well as one representant of the ST131, a highly virulent multidrug-resistant pathogen.

Bactericidal activity of tailocins was detected as zone of clearing on the bacterial lawn. Two results were recorded: i) the maximal level of dilution still displaying a bactericidal activity, ii) the turbidity of clearing zone, which was annotated from 1 (turbid) to 5 (clear).

**Table 4** and **Table 5** below show the activity of selected tailocins on enteropathogenic and uropathogenic *E. coli* described above. All tested tailocins demonstrated a zone of clearing on at least one strain, even diluted at 10⁻⁴ even 10⁻⁵, suggesting that nanograms of tailocins can generate bactericidal activity. As for phages, from which they derive, the killing spectrum of each tailocin is relatively narrow. Some target bacterial strains displayed a rather susceptible phenotype such as EC7 or EC13, while some others were clearly more resistant (i.e. EC107 and EC108).

Tailocins with the broadest spectrum are those produced by strains Ps6, Ps36, Ps38, Ps53, andPs54, which targeted 21, 16, 21, 20, 18 on the 28 *E. coli* tested, respectively. On the opposite, tailocins produced by strain S19 were only active on 3 *E. coli* strains.

Narrow spectrum agents that act against specific strains and do not generate resistance in other pathogens have become an attractive alternative to perform highly targeted treatment preserving patient's entire microbiome. In contrast, as for phages, the utilization of cocktails composed by tailocins with complementary bactericidal activity spectrum will allow to ensure a broad spectrum of action and probably to prevent or minimize bacterial resistance of the targets.

Among the 15 described tailocins, all showed an activity against at least one strain of enteropathogenic *E. coli* and/or strains isolated from faeces. The tailocins produced by *P. fluorescens* strain Ps36 notably targeted EHEC, with a lethal activity on the O157:H7 strains as well as the strain displaying the O26 serotype.

On the other hand, the 15 tailocins demonstrated activity on at least 2 UPEC among the 29 tested. Killing spectrum was not associated with phylogroup, serotype, origin or infection site of the UPEC targets. Tested tailocins were actives against bacterial targets belonging to the 4 main phylogroups and were able to efficiently kill antibiotic resistant bacteria even the well know epidemic and multi-drug resistant ST131 clone. The tailocins produced by strains Ps6, Ps36, Ps38, Ps53and Ps54 clearly displayed the broadest spectrum and the strongest activity against this group of *E. coli.*

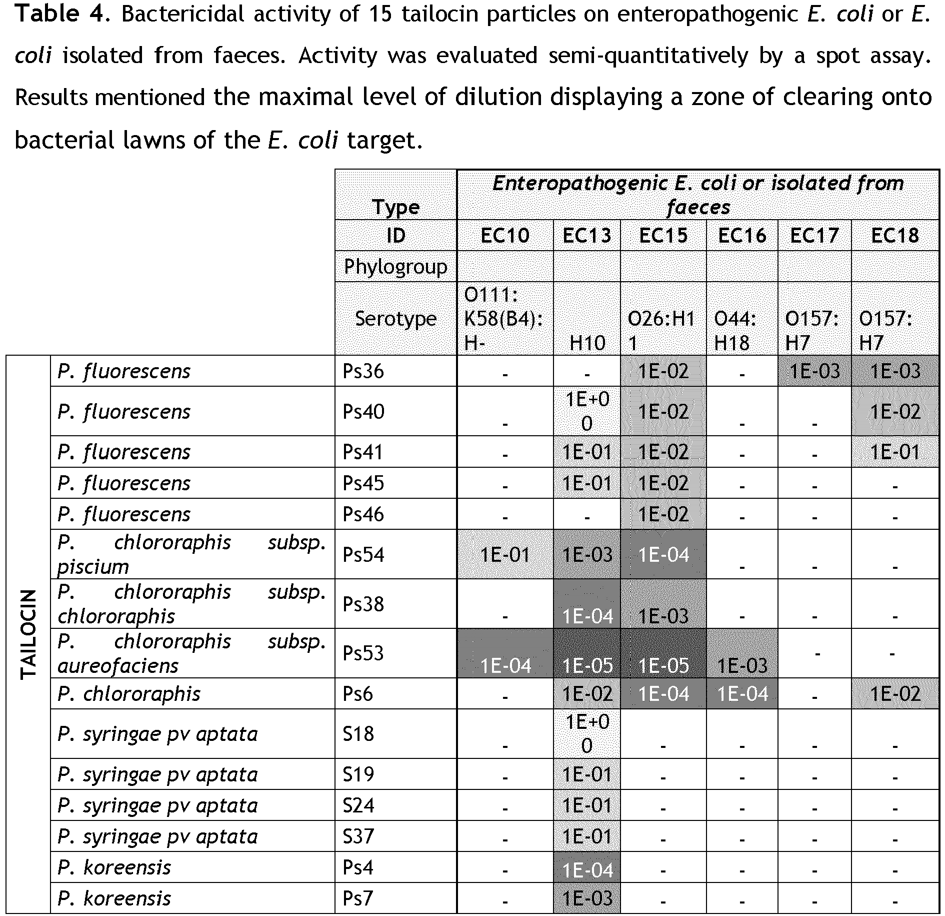

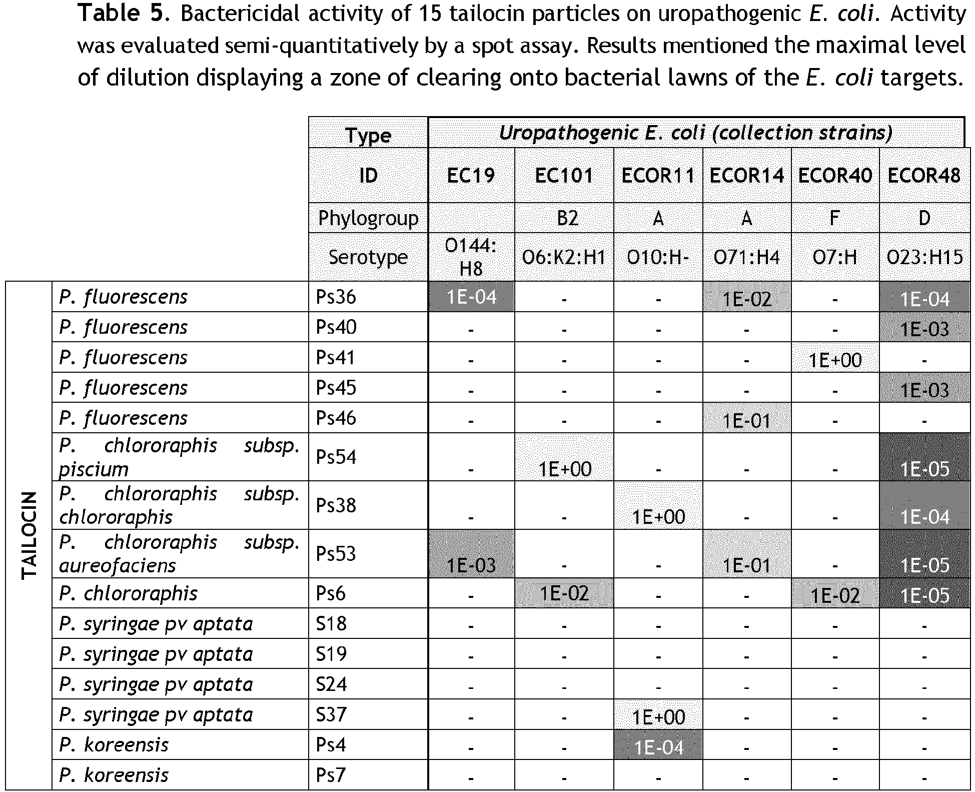

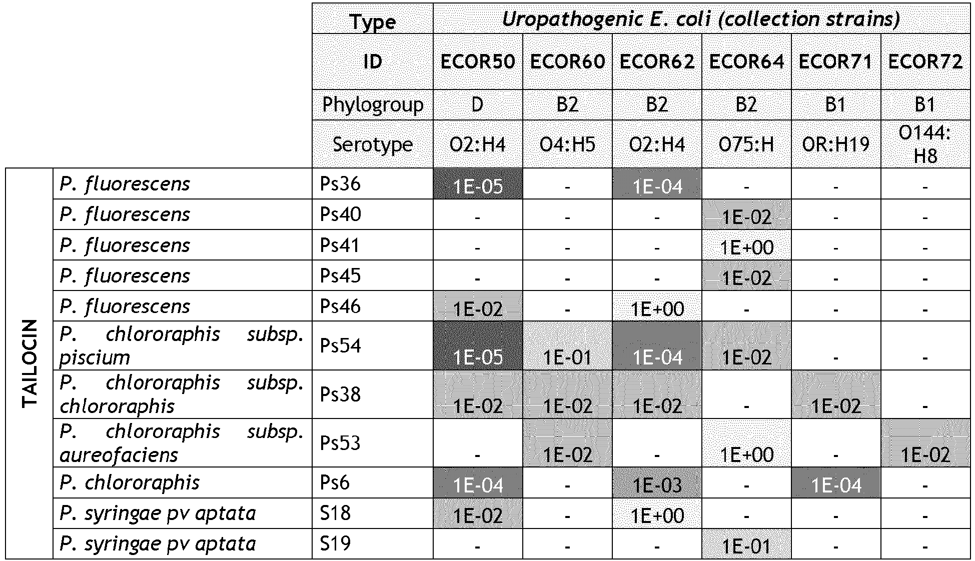

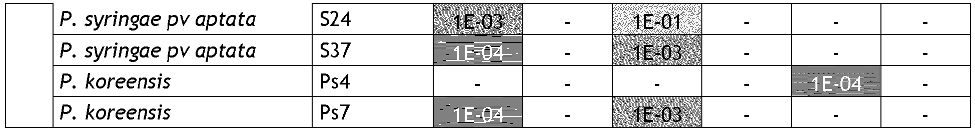

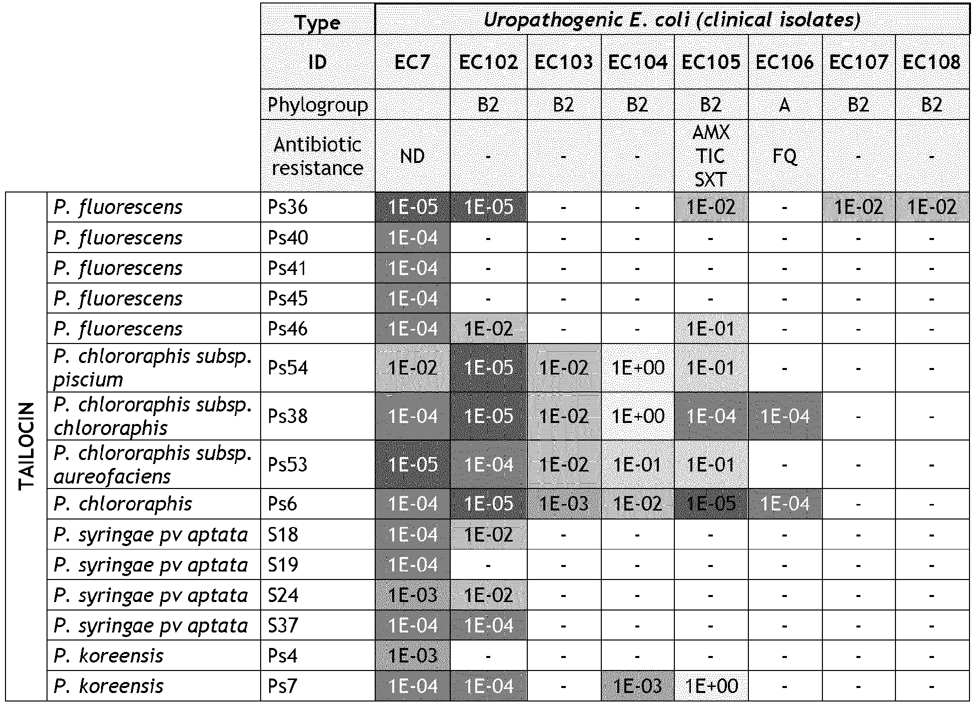

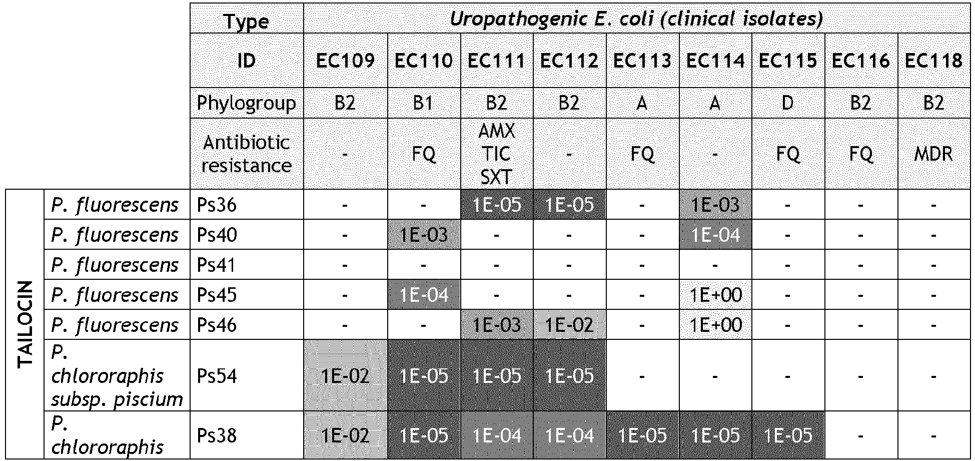

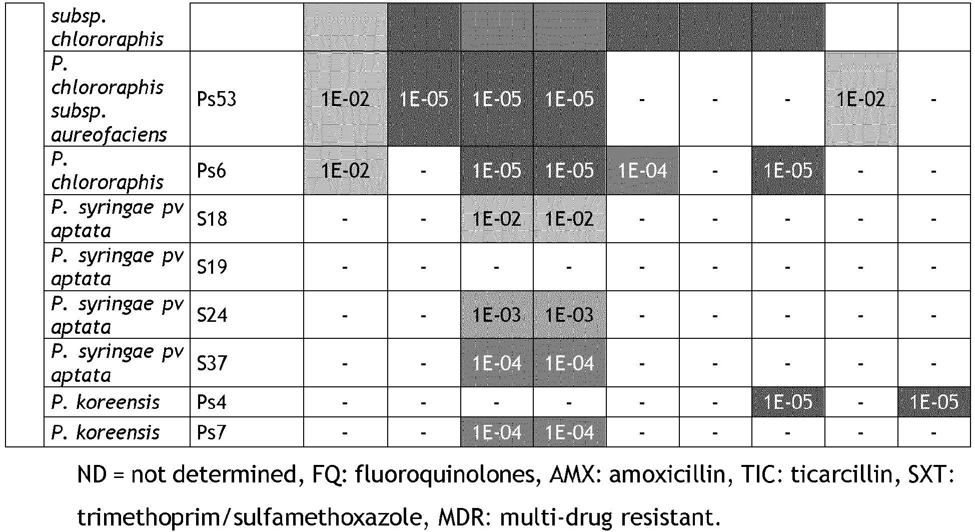

### Combination of tailocins isolated from P. chlororaphis and P. koreensis showed an efficient complementarity to kill drug-resistant UPEC

The antimicrobial properties of a cocktail composed by tailocins produced by *P. chlororaphis* Ps38, *P. chlororaphis* Ps53, and *P. koreensis* Ps4 was screened semi-quantitatively by a dilution method. Serial dilutions of purified lysates of each of the three *Pseudomonas* strains as well as of the tailocin cocktail were spotted onto bacterial lawns of antibiotic-resistant uropathogenic *E. coli.* As described above bactericidal activity of tailocins was detected as zone of clearing on the bacterial lawn.

Tailocin cocktail showed a complete complementarity to kill the drug-resistant UPEC **(Table 6).** This is notably the case against EC110, EC111, EC115, EC116 and EC118, on which the cocktail showed an activity equivalent to the highest maximal level of dilution displayed by the best tailocin tested individually. A minor effect of the dilution of tailocins in the cocktail was observed on EC105 and EC113. Furthermore, the tailocin cocktail applied to EC106 exhibited a 2-log reduction in activity compared to TC-Ps38 alone suggesting an antagonistic effect inside the cocktail in presence of this strain, specifically. Nevertheless, this do not prevent the complementarity properties displayed by the cocktail against all the selected targets.

While offering a targeted spectrum dedicated to antibiotic-resistant strains, the tailocin cocktail also showed a strong bactericidal activity against the multi-drug resistant and highly virulent ST131 strain (i.e. EC118).

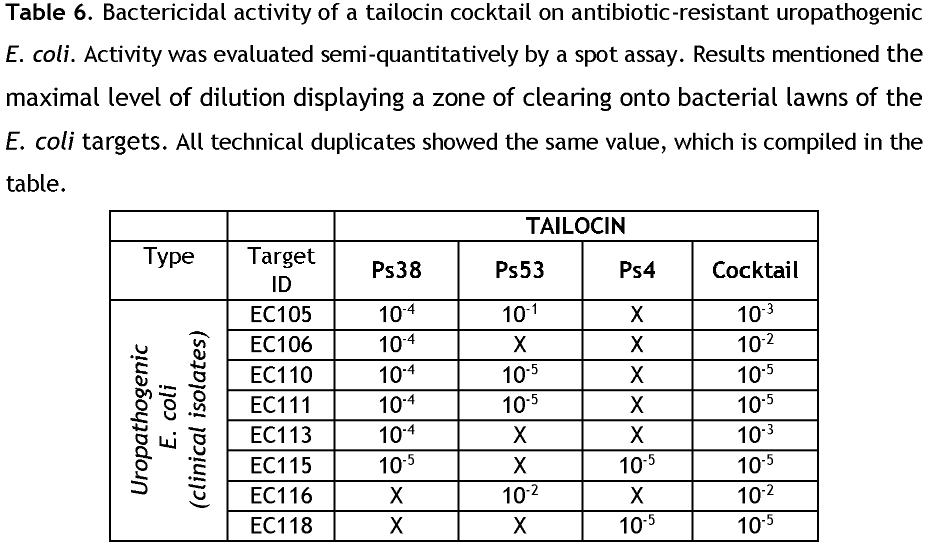

### Conclusions

The above results show that tailocins produced by various *P. fluorescens, P. chlororaphis, P. koreensis* and *P.* syringae species surprisingly show inter-order bactericidal activity against various enteropathogenic or uropathogenic *E. coli* strains.

While each tailocin has some specificity for a subgroup of enteropathogenic or uropathogenic *E. coli* strains, some tailocins have a broader activity spectrum than others. It is especially the case of the combination of two tailocins produced by *P. fluorescens* strain Ps36. In addition, a broad-spectrum bactericidal activity against enteropathogenic or uropathogenic *E. coli* strains can be obtained by combining tailocins produced by several *P. fluorescens, P. chlororaphis, P. koreensis* and *P.* syringae species. In addition, more narrow-spectrum bactericidal activities against particular sub-groups of *E. coli -* such as antibiotic-resistant uropathogenic *E. coli* - can be achieved by generating dedicated alternative tailocin combinations.

### Example 2: Identification and characterization of a cluster encoding two tailocins in Pseudomonas fluorescens strain Ps36

As *Pseudomonas fluorescens* strain Ps36 showed particularly broad-spectrum and high efficiency bactericidal activity against a variety of enteropathogenic and uropathogenic *E. coli* strains, the tailocins produced by this strain were identified and characterized.

### Materials and Methods

### Visualization of tailocin particles using transmission electron microscopy (TEM).

100 µL of purified tailocin suspension were washed and concentrated by centrifugation (29500 g for 1 h at 4 °C) in NH4-acetate 0.1M. 5 µL of the suspension were then adsorbed on a glow-discharged copper 300 mesh grid coated with formvar (Delta Microscopies) for 2 min at room temperature. Grids were washed with two 10µL-droplets of uranyl acetate 2 % in H2O and then stained by incubation with one 10µL-droplets of uranyl acetate 2 % during 30 sec. Excess of uranyl acetate was drained on a blotting paper and grids were dried for 10 min before image acquisition. Micrographs were taken with a JEOL 1400 Flash 120 kV with a high sensitivity sCMOS JEOL Matataki Flash 2048×2048 px² camera.

### Genome sequencing of Pseudomonas fluorescens Ps36.

Overnight liquid culture of Ps36 was sent to Eurofins Genomics (Germany) for DNA extraction and genome sequencing. Sequencing library was prepared by using the NEBNext Ultra^{™} II FS DNA Library Prep Kit and sequenced via an Illumina TruSeq 2x150bp protocol. Reads quality was evaluated with FastQC 0.11.90 (Andrews, 2010) followed by a cleaning by using PRINSEQ 0.20.4 (Schmieder and Edwards, 2011) and Cutadapt 3.4 (Martin, 2011). Reads were assembled using SPAdes 3.15.4 (Nurk et al., 2013) and contigs inferior to 200-bp were eliminated. Assembly quality was evaluated with Bowtie2 2.4.4 (Langmead and Salzberg, 2012), Samtools 2.02 (Li et al., 2009), BUSCO 4.0.2 (Seppey et al., 2019), and contigs were compared to RefSeq database to validate the purity of sequences. Structural annotation was performed via Prokka 1.14.5 (Seemann, 2014) and associated software (Uniprot KB (UniProt Consortium, 2019), Bacterial Antimicrobial Resistance Reference Gene Database (NIH, 2016), Barrnap 0.9 (Seemann, 2019), Prodigal 2.6.3 (Hyatt et al., 2010)). Prophage sequences were predicted by using Vibrant 1.2.1 (Kieft et al., 2020), PhiSpy 4.2.19 (Akhter et al., 2012), et Phigaro 2.3.0 (Starikova et al., 2019). PhiSpy was runned 3-times par genome by using the default mode, the training mode and the pVOG mode. Active prophage elements were searched by using hafeZ 1.0.2 (Turkington et al., 2021). Regions encoding putative prophages were reannotated by using the rTOOLS2 pipeline of Rime Bioinformatics company.

### Tailocin prediction and genomic comparison.

Whole *P. fluorescens* Ps36 genome sequence was explored for the presence of genetic regions encoded tailocin elements by using Geneious 2023.2.1 (created by Biomatters Ltd., Auckland, New Zealand). The genomic regions of *P. fluorescens* Ps36 immediately surrounding *trpE* and *trpG* as well as *mutS* and *cinAlrecA* genes were manually inspected for, first, the presence of viral sequences, and then, the absence of genes involved in capsid formation.

Syntheny and sequence homology between *Pseudomonas* tailocin gene clusters were evaluated by aligning genomic tailocin hotspot regions (*trpE-trpG* ; *mutS-cinAlrecA*) with Clustal Omega 1.2.2 (mBed algorithm) (Bioinformatics, 4th Edition I Wiley, n.d.) implemented in Geneious 2023.2.1. These alignments were used as support for functional gene annotation and genomic characterization of the two particles encoded by the Ps36 tailocin clusters. Finally, the absence of identity of Ps36 tailocin clusters with additional genomes deposited on the National Center of Biotechnology Information (NCBI) database were validated by MegaBlast. And, the absence of any other tailocin elements in the genome of *P. fluorescens* Ps36 was evaluated by a manual inspection of all putative prophage sequences predicted by the bioinformatic tool PHASTER (Zhou et al., 2011; Arndt et al., 2016).

### Results

### Characterization of the P. fluorescens strain Ps36 genome reveals an original gene cluster encoding two tailocins

The R- and F-type tailocin gene clusters are frequently found between the *trpE* and *trpG* genes, or between the *mutS* and *recA*/*cinA* genes in *Pseudomonas* (Ghequire and Mot, 2015). To locate these genomic hotspots, *P. fluorescens* strain Ps36 was submitted to genome sequencing. After assembly and annotation (see **Table 7**), the two regions were extracted and analysed for the presence of gene clusters encoding tailocins.

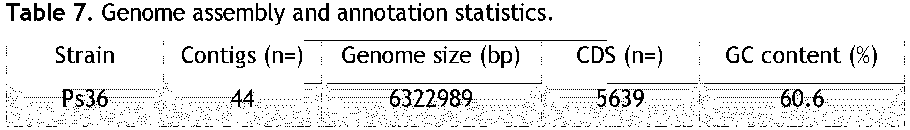

While the genomic region between the *trpE* and *trpG* genes does not contain any cluster, the region between the *mutS* and *cinA* genes was found to encode a tailocin gene cluster of 36077-bp. This gene cluster contains 44 putative CDS encompassing predicted functions characteristic of tailocin gene clusters (i.e. tail assembly, regulation, lysis) and lacking genes involved in capsid formation (see **Table 8**).

Alignments with tailocin-related regions described in literature and available in sequence databases showed that Ps36 tailocin gene cluster consists of two different tailocin units (see **Figure 1** and **Table 8**).

The presence of a single set of regulatory and lysis genes flanking this mosaic region strongly suggests that the tailocin pair is released in a coordinated fashion. According to the review of Maarten G.K. Ghequire and René De Mot (Ghequire and Mot, 2015), the two different tailocin units were annotated by type : i) fluorescin type for TC1-Ps36, ii) syringacin type for TC2-Ps36.

Finally, no additional putative tailocin gene cluster was identified in the rest of the genome via a manual inspection of prophage sequences predicted by PHASTER tool.

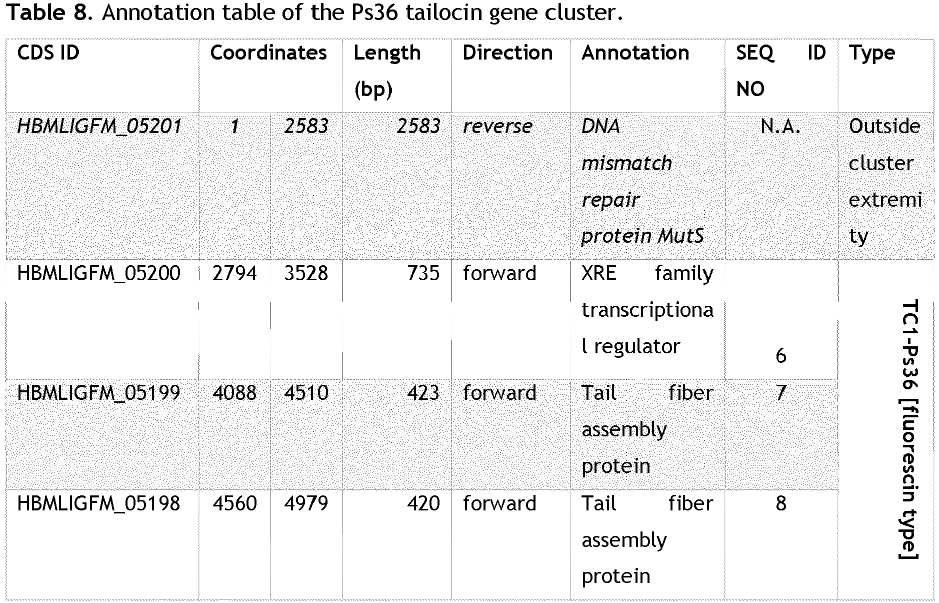

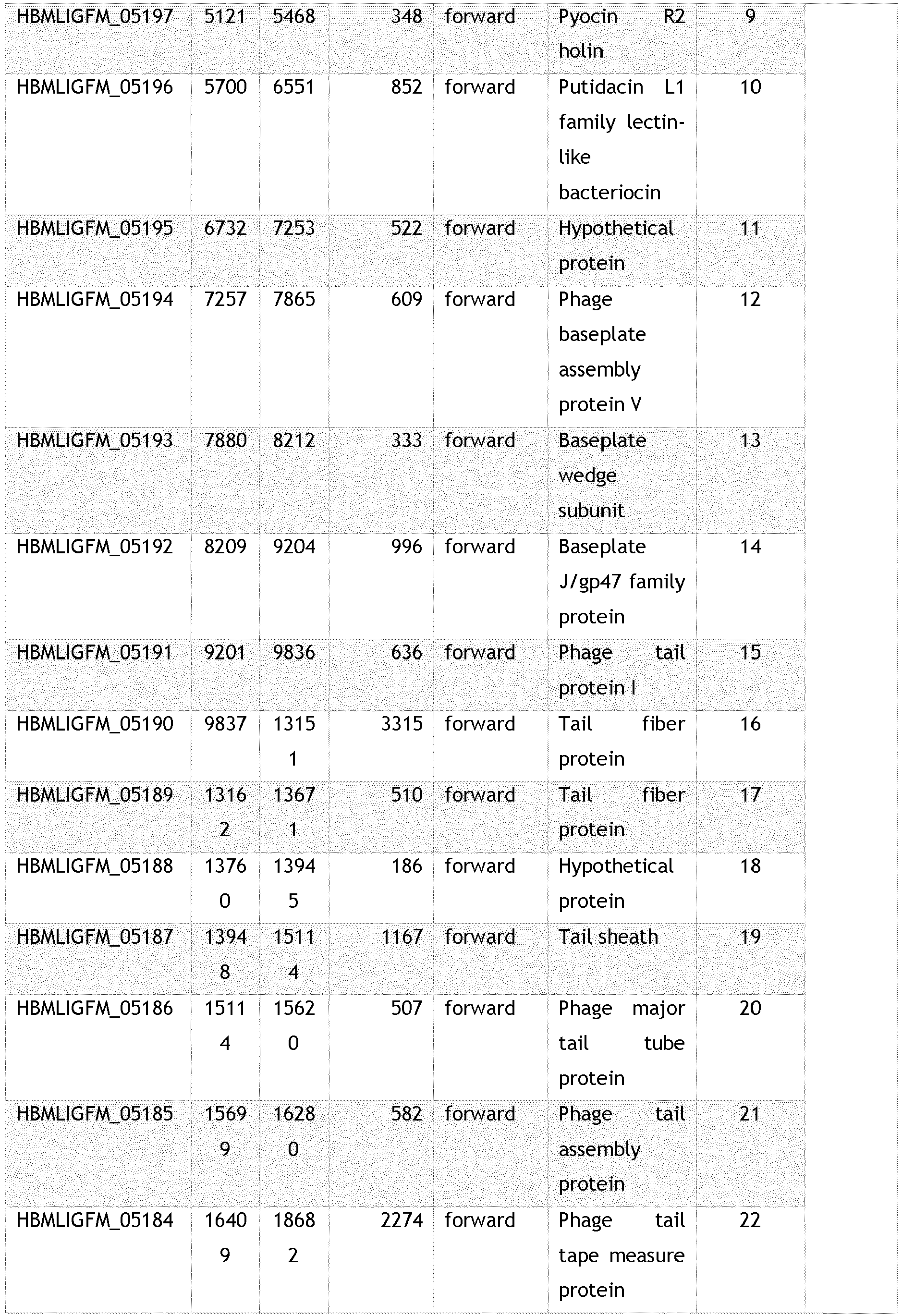

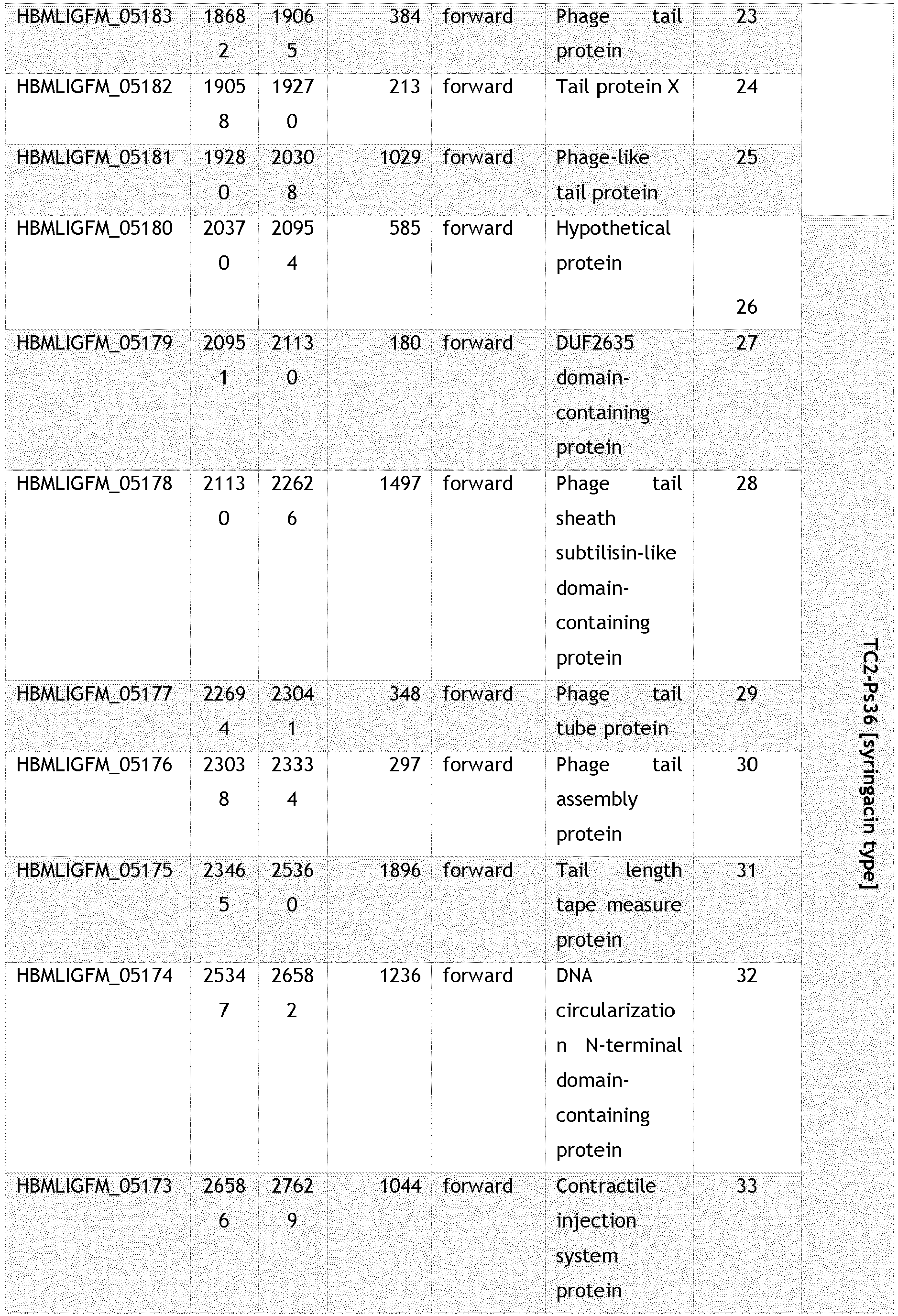

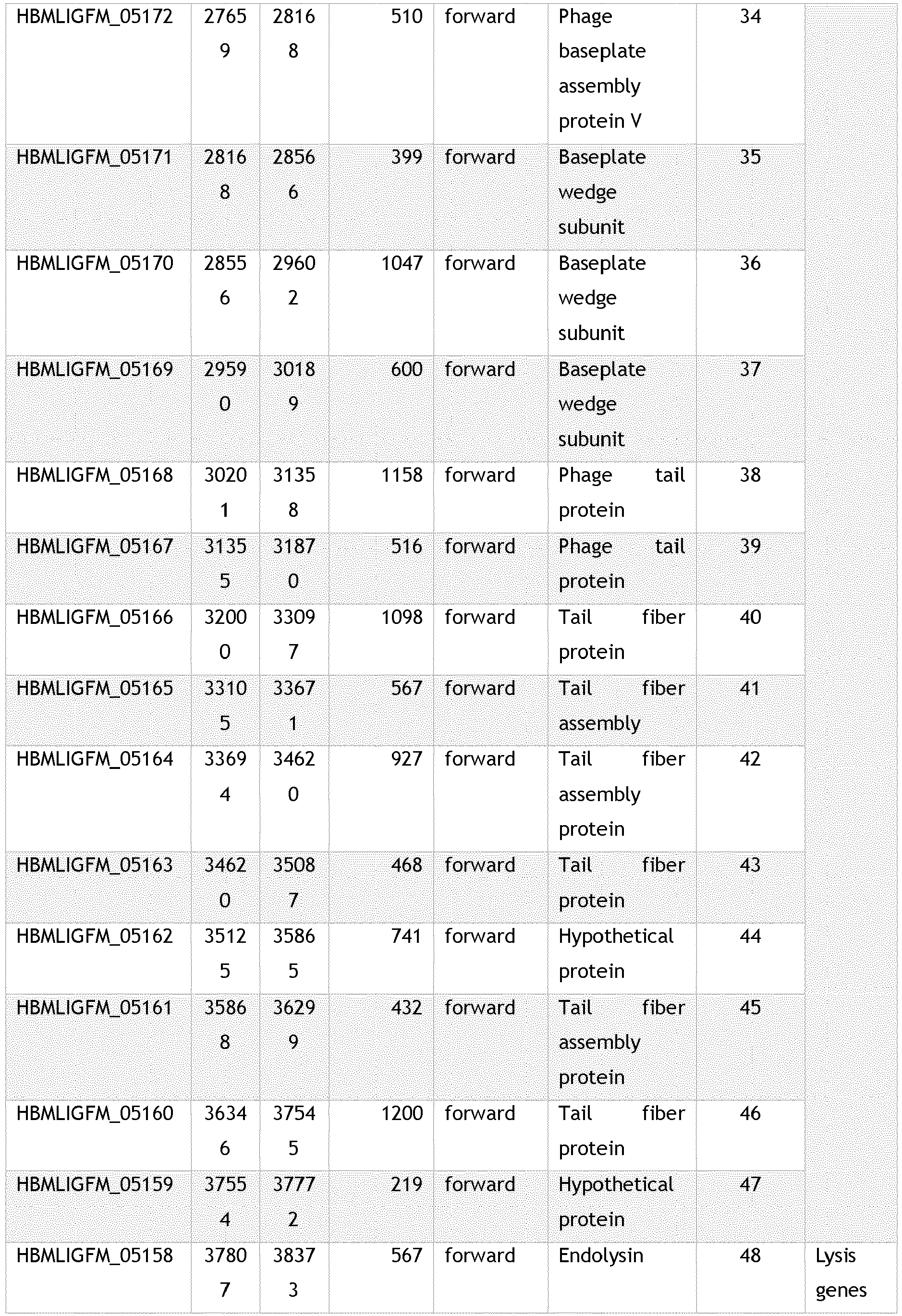

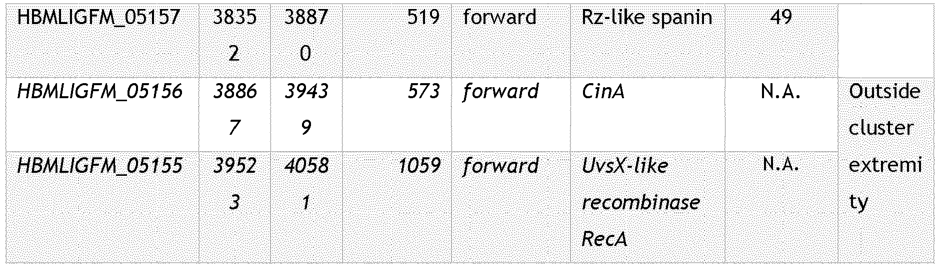

Originality of these two tailocin units as well as the whole cluster was evaluated by comparison with tailocin-related regions described in literature and available in sequence databases. The maximum of sequence identity observed with known clusters was 77% and was found with the nucleotide sequence of the syringacin-type tailocin from *Pseudomonas fluorescens* strain Pf0-1 **(Table 9),** demonstrating that the two tailocin sequences we found in *P. fluorescens* Ps 36 are original entities.

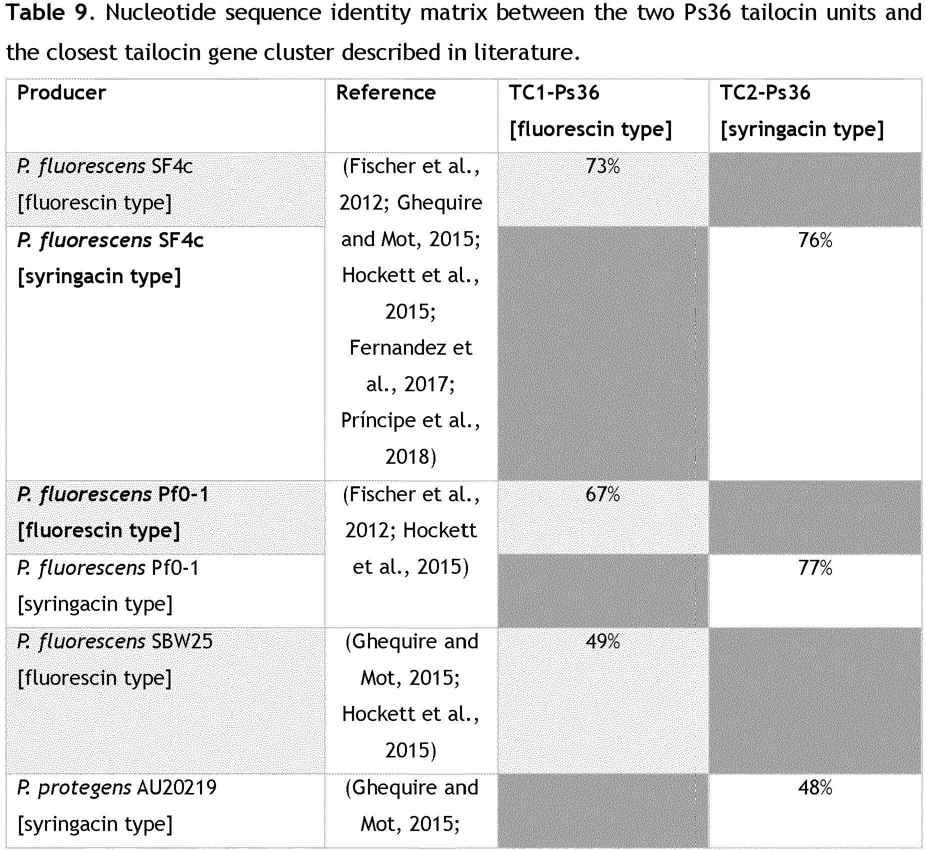

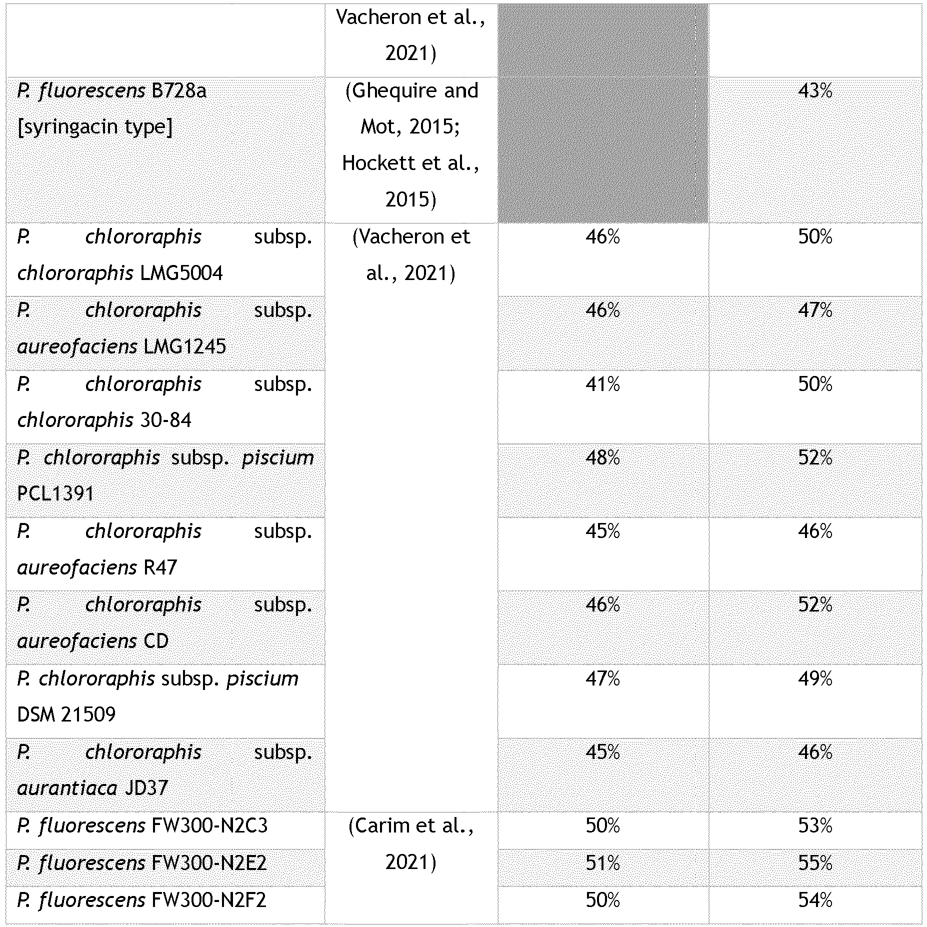

### Electron microscopy analysis of the Ps36 purified lysate suggested the presence of two types of particles

Tailocins were obtained from *P. fluorescens* Ps36 by mitomycin C induction, and the structure of the purified particles was analysed by transmission electron microscopy (TEM).

Microscopic images revealed the presence of a large number of phage tail-like bacteriocins **(****Figure 2A****).** The dimensions of the tailocins were determined from TEM images **(****Figure 2D****).** Measurements obtained from 92 tailocin particles revealed the presence of a very large majority population of homogeneous R-type particles and some heterogeneous particles. The first population, by including 83.7% of the total particles, was clearly majority. It was composed by homogeneous particles characterized by an average length and diameter of approximately 118 ± 16 nm and 19 ± 2 nm, respectively **(****Figure 2B****).** These measurements were in agreement with the values reported for R-pyocin from *P. aeruginosa* and some other tailocins (Fernandez et al., 2017). In contrast, the second population is particularly heterogeneous in length, with irregular-length tubes attached to a baseplate with associated tail fibres, measuring up to 466 nm **(****Figure 2C****).**

### Characterization of Ps36 tailocins bactericidal specificity on E. coli

Ps36 purified tailocin preparations were used to test their killing effects on log-phase cultures of two target strains, one representative of uropathogenic and another of enteropathogenic *E. coli.*

Serial dilutions of the Ps36 purified tailocins were spotted onto soft agar overlays inoculated with target bacterial strains. Bactericidal activities were detected as zones of clearing on the bacterial lawn. **Figure 3A** shows typical zones of inhibition obtained with tailocins, for which clearing gradually disappeared along serial dilutions without the appearance of plaque-forming units, characteristics of bacteriophages. These overlay assays revealed that a few nanograms of Ps36 purified extracts were enough to kill populations of EC7 and EC15 target bacteria. The absence of bacterial survival after hours of incubation strongly suggested that Ps36 tailocins exhibit a strong bactericidal activity. In addition, Ps36 tailocins demonstrated strong activity on a wide panel of *E. coli,* both enteropathogenic and uropathogenic strains, with an activity on relevant strains in these groups (see **Table 4** and **Table 5**).

Furthermore, MBC assays were conducted on EC7 and EC15 strains in order to determine the potency of the Ps36 purified extracts to reduce the viability of uropathogenic and enteropathogenic *E. coli.* Purified TC-Ps36 killed EC7 with a nanomolar MBC and EC15 with a micromolar MBC **(****Figure 3B****),** demonstrating that low quantities of TC-Ps36 are enough to definitively eliminate bacterial targets. These results confirm the strong bactericidal activity visualized by spot assay.

### Conclusions

The above results confirm that *P. fluorescens* strain Ps36 produces two distinct tailocins, one fluorescin-type (TC1) and one syringacin-type (TC2), encoded by two distinct units of a tailocin cluster between the *mutS* and *cinA* genes.

They further show that the nucleotide sequence of the tailocin cluster of *P. fluorescens* strain Ps36 has limited homology to known tailocin clusters of other *Pseudomonas* species. The original sequence of the tailocin cluster of *P. fluorescens* strain Ps36 might explain its particularly broad-spectrum bactericidal activity against enteropathogenic and uropathogenic *E. coli* strains.

Finally, the above results confirm that the tailocins produced by *P. fluorescens* strain Ps36 show bactericidal activity against enteropathogenic and uropathogenic *E. coli* strains at low concentration, further supporting the interest of these particular tailocins.

### BIBLIOGRAPHIC REFERENCES

Akhter, S., Aziz, R. K., and Edwards, R. A. (2012). PhiSpy: a novel algorithm for finding prophages in bacterial genomes that combines similarity- and composition-based strategies. Nucleic Acids Res 40, e126. doi: 10.1093/nar/gks406.
Alharbi, M. G., Al-Hindi, R. R., Esmael, A., Alotibi, I. A., Azhari, S. A., Alseghayer, M. S., et al.
(2022). The "Big Six": Hidden Emerging Foodborne Bacterial Pathogens. Trop Med Infect Dis 7, 356. doi: 10.3390/tropicalmed7110356.
Alzohairy A. 2011. BioEdit: An important software for molecular biology. GERF Bulletin of Biosciences, 2, 60-61Amor, K., Heinrichs, D. E., Frirdich, E., Ziebell, K., Johnson, R. P., and Whitfield, C. (2000). Distribution of Core Oligosaccharide Types in Lipopolysaccharides from Escherichia coli. Infect Immun 68, 1116-1124.
Andrews, S. (2010). FastQC: a quality control tool for high throughput sequence data. Available online at: http://www.bioinformatics.babraham.ac.uk/projects/fastqc.
Arndt, D., Grant, J. R., Marcu, A., Sajed, T., Pon, A., Liang, Y., et al. (2016). PHASTER: a better, faster version of the PHAST phage search tool. Nucleic Acids Res 44, W16-21. doi: 10.1093/nar/gkw387.
Bielaszewska, M., Zhang, W., Mellmann, A., and Karch, H. (2007). Enterohaemorrhagic Escherichia coli O26:H11/H-: a human pathogen in emergence. Berl Munch Tierarztl Wochenschr 120, 279-287.
Bioinformatics, 4th Edition | Wiley (n.d.). Wiley.com. Available at: https://www.wiley.com/en-us/Bioinformatics%2C+4th+Edition-p-9781119335580 [Accessed December 1, 2023].
Bumunang, E. W., Zaheer, R., Niu, D., Narvaez-Bravo, C., Alexander, T., McAllister, T. A., et al.
(2023). Bacteriophages for the Targeted Control of Foodborne Pathogens. Foods 12, 2734. doi: 10.3390/foods12142734.
Caméléna, F., Birgy, A., Smail, Y., Courroux, C., Mariani-Kurkdjian, P., Le Hello, S., et al. (2019). Rapid and Simple Universal Escherichia coli Genotyping Method Based on Multiple-Locus Variable-Number Tandem-Repeat Analysis Using Single-Tube Multiplex PCR and Standard Gel Electrophoresis. Appl Environ Microbiol 85, e02812-18. doi: 10.1128/AEM.02812-18.
Carim, S., Azadeh, A. L., Kazakov, A. E., Price, M. N., Walian, P. J., Lui, L. M., et al. (2021). Systematic discovery of pseudomonad genetic factors involved in sensitivity to tailocins. ISME J 15, 2289-2305. doi: 10.1038/s41396-021-00921-1.
Croxen MA, Law RJ, Scholz R, Keeney KM, Wlodarska M, Finlay BB. Recent advances in understanding enteric pathogenic Escherichia coli. Clin Microbiol Rev. 2013 Oct;26(4):822-80. doi: 10.1128/CMR.00022-13. Erickson MC, Doyle MP. Food as a vehicle for transmission of Shiga toxin-producing Escherichia coli. J Food Prot. 2007 Oct;70(10):2426-49. doi: 10.4315/0362-028x-70.10.2426.
Fazly Bazzaz, B. S., Darvishi Fork, S., Ahmadi, R., and Khameneh, B. (2021). Deep insights into urinary tract infections and effective natural remedies. African Journal of Urology 27, 6. doi: 10.1186/s12301-020-00111-z.
Fernandez, M., Godino, A., Principe, A., Morales, G. M., and Fischer, S. (2017). Effect of a Pseudomonas fluorescens tailocin against phytopathogenic Xanthomonas observed by atomic force microscopy. J. Biotechnol. 256, 13-20. doi: 10.1016/j.jbiotec.2017.07.002.
Fischer, S., Godino, A., Quesada, J. M., Cordero, P., Jofré, E., Mori, G., et al. (2012). Characterization of a phage-like pyocin from the plant growth-promoting rhizobacterium Pseudomonas fluorescens SF4c. Microbiology 158, 1493-1503. doi: 10.1099/mic.0.056002-0.
Flores-Mireles AL, Walker JN, Caparon M, Hultgren SJ. Urinary tract infections: epidemiology, mechanisms of infection and treatment options. Nat Rev Microbiol. 2015 May;13(5):269-84. doi: 10.1038/nrmicro3432. Ghequire, M. G. K., and Mot, R. D. (2015). The Tailocin Tale: Peeling off Phage Tails. Trends in Microbiology 23, 587-590. doi: 10.1016/j.tim.2015.07.011.
Guzman LM, Belin D, Carson MJ, Beckwith J. Tight regulation, modulation, and high-level expression by vectors containing the arabinose PBAD promoter. J Bacteriol. 1995 Jul; 177(14):4121 - 30. doi: 10.1128/jb.177.14.4121-4130.1995. Hockett, K. L., Renner, T., and Baltrus, D. A. (2015). Independent Co-Option of a Tailed Bacteriophage into a Killing Complex in Pseudomonas. mBio 6. doi: 10.1128/mBio.00452-15.
Hyatt, D., Chen, G.-L., Locascio, P. F., Land, M. L., Larimer, F. W., and Hauser, L. J. (2010). Prodigal: prokaryotic gene recognition and translation initiation site identification. BMC Bioinformatics 11, 119. doi: 10.1186/1471-2105-11-119.
Kaper, J. B., Nataro, J. P., and Mobley, H. L. T. (2004). Pathogenic Escherichia coli. Nat Rev Microbiol 2, 123-140. doi: 10.1038/nrmicro818.
Kieft, K., Zhou, Z., and Anantharaman, K. (2020). VIBRANT: automated recovery, annotation and curation of microbial viruses, and evaluation of viral community function from genomic sequences. Microbiome 8, 90. doi: 10.1186/s40168-020-00867-0.
Kot B. Antibiotic Resistance Among Uropathogenic Escherichia coli. Pol J Microbiol. 2019 Dec;68(4):403-415. doi: 10.33073/pjm-2019-048. Langmead, B., and Salzberg, S. L. (2012). Fast gapped-read alignment with Bowtie 2. Not Methods 9, 357-359. doi: 10.1038/nmeth.1923.
Lavigne JP, Vergunst AC, Goret L, Sotto A, Combescure C, Blanco J, O'Callaghan D, Nicolas-Chanoine MH. Virulence potential and genomic mapping of the worldwide clone Escherichia coli ST131. PLoS One. 2012;7(3):e34294. doi: 10.1371/journal.pone.0034294.Li, H., Handsaker, B., Wysoker, A., Fennell, T., Ruan, J., Homer, N., et al. (2009). The Sequence Alignment/Map format and SAMtools. Bioinformatics 25, 2078-2079. doi: 10.1093/bioinformatics/btp352.
Loubet P, Ranfaing J, Dinh A, Dunyach-Remy C, Bernard L, Bruyère F, Lavigne JP, Sotto A. Alternative Therapeutic Options to Antibiotics for the Treatment of Urinary Tract Infections. Front Microbiol. 2020 Jul 3;11:1509. doi: 10.3389/fmicb.2020.01509. Luo, C., Hu, G.-Q., and Zhu, H. (2009). Genome reannotation of Escherichia coli CFT073 with new insights into virulence. BMC Genomics 10, 552. doi: 10.1186/1471-2164-10-552.
Martin, M. (2011). Cutadapt removes adapter sequences from high-throughput sequencing reads. EMBnet.journal 17, 10-12. doi: 10.14806/ej.17.1.200.
Mierendorf RC, Morris BB, Hammer B, Novy RE. Expression and Purification of Recombinant Proteins Using the pET System. Methods Mol Med. 1998;13:257-92. doi: 10.1385/0-89603-485-2:257.
Munkhdelger Y, Gunregjav N, Dorjpurev A, Juniichiro N, Sarantuya J. Detection of virulence genes, phylogenetic group and antibiotic resistance of uropathogenic Escherichia coli in Mongolia. J Infect Dev Ctries. 2017 Jan 30;11(1):51-57. doi: 10.3855/jidc.7903. Needleman et Wunsch. J.Mol. Biol. 48,443-453, 1970.
Nicolas-Chanoine MH, Bertrand X, Madec JY. Escherichia coli ST131, an intriguing clonal group. Clin Microbiol Rev. 2014 Jul;27(3):543-74. doi: 10.1128/CMR.00125-13. NIH (2016). Bacterial Antimicrobial Resistance Reference Gene Database. Available at: https://www.ncbi.nlm.nih.gov/bioproject/313047,.
Nurk, S., Bankevich, A., Antipov, D., Gurevich, A. A., Korobeynikov, A., Lapidus, A., et al. (2013). Assembling single-cell genomes and mini-metagenomes from chimeric MDA products. J Comput Biol 20, 714-737. doi: 10.1089/cmb.2013.0084.
Ochman, H., and Selander, R. K. (1984). Standard reference strains of Escherichia coli from natural populations. J Bacterial 157, 690-693.
Patel, I. R., Gangiredla, J., Mammel, M. K., Lampel, K. A., Elkins, C. A., and Lacher, D. W. (2018). Draft Genome Sequences of the Escherichia coli Reference (ECOR) Collection. Microbiol Resour Announc 7, e01133-18. doi: 10.1128/MRA.01133-18.
Picard B, Garcia JS, Gouriou S, Duriez P, Brahimi N, Bingen E, Elion J, Denamur E. The link between phylogeny and virulence in Escherichia coli extraintestinal infection. Infect Immun. 1999 Feb;67(2):546-53. doi: 10.1128/IAI.67.2.546-553.1999.
Poirel, L., Madec, J.-Y., Lupo, A., Schink, A.-K., Kieffer, N., Nordmann, P., et al. (2018). Antimicrobial Resistance in Escherichia coli. Microbiology Spectrum 6, 10.1128/microbiolspec.arba-0026-2017. doi: 10.1128/microbiolspec.arba-0026-2017.
Principe, A., Fernandez, M., Torasso, M., Godino, A., and Fischer, S. (2018). Effectiveness of tailocins produced by Pseudomonas fluorescens SF4c in controlling the bacterial-spot disease in tomatoes caused by Xanthomonas vesicatoria. Microbiol. Res. 212-213, 94-102. doi: 10.1016/j.micres.2018.05.010.
Reid, S. D., Betting, D. J., and Whittam, T. S. (1999). Molecular Detection and Identification of Intimin Alleles in Pathogenic Escherichia coli by Multiplex PCR. J Clin Microbiol 37, 2719-2722.
Schmieder, R., and Edwards, R. (2011). Quality control and preprocessing of metagenomic datasets. Bioinformatics 27, 863-864. doi: 10.1093/bioinformatics/btr026.
Seemann, T. (2014). Prokka: rapid prokaryotic genome annotation. Bioinformatics 30, 2068-2069. doi: 10.1093/bioinformatics/btu153.
Seemann, T. (2019). Barrnap.
Seppey, M., Manni, M., and Zdobnov, E. M. (2019). BUSCO: Assessing Genome Assembly and Annotation Completeness. Methods Mol Biol 1962, 227-245. doi: 10.1007/978-1-4939-9173-0_14. Sheerin, N. S. (2011). Urinary tract infection. Medicine 39, 384-389. doi: 10.1016/j. mpmed.2011.04.003.
Starikova, E., O. Tikhonova, P., A. Prianichnikov, N., Rands, C., M. Zdobnov, E., and M. Govorun, V. (2019). Phigaro: high throughput prophage sequence annotation. doi: 10.1101/598243.
Studier FW, Rosenberg AH, Dunn JJ, Dubendorff JW. Use of T7 RNA polymerase to direct expression of cloned genes. Methods Enzymol. 1990;185:60-89. doi: 10.1016/0076-6879(90)85008-c. Tartof SY, Solberg OD, Manges AR, Riley LW 2005. Analysis of a Uropathogenic Escherichia coli Clonal Group by Multilocus Sequence Typing. J Clin Microbiol43:. https://doi.org/10.1128/jcm.43.12.5860-5864.2005.
Turkington, C. J. R., Abadi, N. N., Edwards, R. A., and Grasis, J. A. (2021). hafeZ: Active prophage identification through read mapping. 2021.07.21.453177. doi: 10.1101/2021.07.21.453177.
UniProt Consortium (2019). UniProt: a worldwide hub of protein knowledge. Nucleic Acids Res 47, D506-D515. doi: 10.1093/nar/gky1049.
Vacheron, J., Heiman, C. M., and Keel, C. (2021). Live cell dynamics of production, explosive release and killing activity of phage tail-like weapons for Pseudomonas kin exclusion. Communications Biology 4, 1-14. doi: 10.1038/s42003-020-01581-1.
Weaver, S. L., Zhu, L., Ravishankar, S., Clark, M., and Baltrus, D. A. (2022). Interspecies Killing Activity of Pseudomonas syringae Tailocins. Microbiology doi: 10.1101/2022.07.29.502058.
Yao, G. W., Duarte, I., Le, T. T., Carmody, L., LiPuma, J. J., Young, R., et al. (2017). A Broad-Host-Range Tailocin from Burkholderia cenocepacia. Applied and Environmental Microbiology 83, e03414-16. doi: 10.1128/AEM.03414-16.
Zhou, Y., Liang, Y., Lynch, K. H., Dennis, J. J., and Wishart, D. S. (2011). PHAST: a fast phage search tool. Nucleic Acids Res 39, W347-352. doi: 10.1093/nar/gkr485.

## Claims

1. A composition comprising a cell lysate, wherein the cell produces at least one tailocin from a *Pseudomonas* bacterium selected from the group consisting of *Pseudomonas fluorescens, Pseudomonas chlororaphis, Pseudomonas koreensis* and *Pseudomonas syringae* species, for use in the prevention or the treatment in a subject of a contamination or an infection by an *Escherichia coli* strain, preferably selected from the group consisting of urogenital infection or gastrointestinal infection.

2. Use of a composition comprising a cell lysate, wherein the cell produces at least one tailocin from a *Pseudomonas* bacterium selected from the group consisting of *Pseudomonas fluorescens, Pseudomonas chlororaphis, Pseudomonas koreensis* and *Pseudomonas syringae* species, for the prevention and/or the treatment of a contamination by an *Escherichia coli* strain in a food product or on a surface, wherein the surface is not a living body part of an animal or human subject.

3. The composition for use according to claim 1 or the use according to claim 2, wherein the cell is a *Pseudomonas* bacterium selected from the group consisting of *Pseudomonas fluorescens, Pseudomonas chlororaphis, Pseudomonas koreensis* and *Pseudomonas syringae* species producing produces at least one tailocin, preferably the *Pseudomonas* bacterium is selected from the group consisting of:
(a) *Pseudomonas fluorescens* strain Ps36 deposited at Collection Nationale de Cultures de Microorganismes (CNCM) on the 21^{st} of March 2024 under accession number I-6068,
(b) *Pseudomonas fluorescens* strain Ps40 available at Collection francaise des bactéries associées aux plantes (CFBP) under accession number 3353;
(c) *Pseudomonas fluorescens* strain Ps41 available at CFBP under accession number 4560;
(d) *Pseudomonas fluorescens* strain Ps45 available at CFBP under accession number 5900;
(e) *Pseudomonas fluorescens* strain Ps46 available at CFBP under accession number 5916;
(f) *Pseudomonas chlororaphis subsp. piscium* strain Ps54 available at Deutsche Sammlung von Mikroorganismen und Zellkulturen (DSMZ) under accession number DSM 21509;
(g) *Pseudomonas chlororaphis subsp. chlororaphis* strain Ps38 available at Deutsche Sammlung von Mikroorganismen und Zellkulturen (DSMZ) under accession number DSM 50083;
(h) *Pseudomonas chlororaphis subsp. aureofaciens* strain Ps53 available at Deutsche Sammlung von Mikroorganismen und Zellkulturen (DSMZ) under accession number DSM 6698;
(i) *Pseudomonas chlororaphis* strain Ps6 deposited at CNCM on the 21^{st} of March 2024 under accession number I-6066;
(j) *Pseudomonas syringae pv aptata* strain S18 available at CFBP under accession number 5471;
(k) *Pseudomonas syringae pv aptata* strain S19 available at CFBP under accession number 5468;
(l) *Pseudomonas syringae pv aptata* strain S24 available at CFBP under accession number 3313;
(m) *Pseudomonas syringae pv aptata* strain S37 available at CFBP under accession number 5429;
(n) *Pseudomonas koreensis* strain Ps4 deposited at CNCM on the 21^{st} of March 2024 under accession number I-6065;
(o) *Pseudomonas koreensis* strain Ps7 deposited at CNCM on the 21^{st} of March 2024 under accession number I-6067;
(p) A genetically engineered variant of any one of (a) to (o), with higher production of the tailocin(s).

4. The composition for use or the use according to anyone of claims 1 to 3, wherein the cell lysate is purified and enriched in tailocin and has preferably been obtained by a method comprising at least the steps of:
(a) cultivating the cell in a cell culture, preferably under conditions stimulating tailocin production by the cell, preferably tailocin production is stimulated by ultraviolet irradiation, anaerobiotic stress, heat induction or addition of a DNA damaging agent such as mitomycin C, ciprofloxacin, or H₂O₂ to the culture medium,
(b) obtaining a raw cell lysate by continuing the bacterial culture of step (a) until natural lysis of the cells or by lysing the cell culture of step (a) ,
(c) filtering the raw cell lysate of step (b) through a first filter with a pore size between 0,1 and 0,45 µm and collecting the filtrate, and
(d) filtering the filtrate of step (c) through a second filter with a cut-off between 50 and 300 kDa and collecting the retentate, thereby obtaining a purified cell lysate enriched in tailocin.

5. The composition for use or the use according to anyone of claims 1 to 4, which:
(a) comprises a protein concentration of at least 1 ng.mL⁻¹;
(b) shows a bactericidal activity on at least one *E. coli* strain at a concentration of at least 10 mg.mL⁻¹.

6. The composition for use or the use according any one of claims 1 to 5, wherein the *Escherichia coli* strain:
(a) has a serotype selected from the group consisting of O157:H7, O44:H18, O111:K58(B4) and O26:H11;
(b) belongs to phylogroups B2 or D;
(c) belongs to the sequence type ST131; or
(d) any combination of (a) to (c).

7. The composition for use according to anyone of claims 1 to 6, wherein
(a) the composition is an intimate care product intended for use in the prevention of an *Escherichia coli* urogenital infection, in particular an *Escherichia coli* urinary tract infection such as cystitis and pyelonephritis; or
(b) the composition is a food supplement intended for use in the prevention of an *Escherichia coli* gastrointestinal infection.

8. An intimate care product or a food supplement comprising one or more cell lysate(s) as defined in any one of claims 1 to 5.

9. An isolated nucleic acid molecule comprising a nucleotide sequence with:
(a) at least 80% identity with SEQ ID NO:1,
(b) at least 80% identity with SEQ ID NO:2,
(c) at least 80% identity with SEQ ID NO:3, or
(d) at least 80% identity with SEQ ID NO:5.

10. The isolated nucleic acid molecule according to claim 9, which:
(a) comprises at least 80% identity with SEQ ID NO:1 and further comprises open reading frames encoding proteins having at least 80% identity with SEQ ID NO: 6 to 25;
(b) comprises at least 80% identity with SEQ ID NO:2 and further comprises open reading frames encoding proteins having at least 80% identity with SEQ ID NO: 26 to 47;
(c) comprises at least 80% identity with SEQ ID NO:3 and further comprises open reading frames encoding proteins having at least 80% identity with SEQ ID NO: 6 to 47; or
(d) comprises at least 80% identity with SEQ ID NO:5 and further comprises open reading frames encoding proteins having at least 80% identity with SEQ ID NO: 6 to 49.

11. An expression vector comprising the nucleic acid molecule according to claim 9 or claim 10, which is preferably a prokaryotic expression vector such as an episomal vector or a Bacterial Artificial Chromosome (BAC).

12. A cell, preferably a bacterium, comprising a nucleic acid molecule according to claim 9 or claim 10, or an expression vector according to claim 11, preferably the cell is selected from:
(a) a *Pseudomonas fluorescens* Ps36 strain deposited at CNCM on the 21^{st} of March 2024 under accession number I-6068, and
(b) a recombinant cell, preferably a recombinant bacterium, transformed by a nucleic acid molecule according to claim 9 or claim 10, or an expression vector according to claim 11.

13. A cell lysate comprising at least one tailocin obtained from the cell according to claim 12, wherein the cell lysate is preferably enriched in tailocin and obtained by a method comprising at least the steps of:
(a) cultivating the cell according to claim 12 in a cell culture, preferably under conditions stimulating tailocin production by the cell, preferably tailocin production is stimulated by ultraviolet irradiation, anaerobic stress, heat induction or addition of a DNA damaging agent such as mitomycin C, ciprofloxacin, or H₂O₂ to the culture medium,
(b) obtaining a raw cell lysate by continuing the cell culture of step (a) until natural lysis of the cells or lysing the cell culture of step (a),
(c) filtering the cell lysate of step (b) through a first filter with a pore size between 0,1 and 0,45 µm and collecting the filtrate, and
(d) filtering the filtrate of step (c) through a second filter with a cut-off between 50 and 300 kDa and collecting the retentate, thereby obtaining a cell lysate enriched in tailocin.

14. A method for preparing a cell lysate enriched in tailocin comprising at least the steps of:
(a) cultivating the cell according to claim 12 in a cell culture, preferably under conditions stimulating tailocin production by the cell, preferably tailocin production is stimulated by ultraviolet irradiation, anaerobic stress, heat induction or addition of a DNA damaging agent such as mitomycin C, ciprofloxacin, or H₂O₂ to the culture medium,
(b) obtaining a raw cell lysate by continuing the cell culture of step (a) until natural lysis of the cells or lysing the cell culture of step a) to obtain a cell lysate,
(c) filtering the cell lysate of step (b) through a first filter with a pore size between 0,1 and 0,45 µm and collecting the filtrate, and
(d) filtering the filtrate of step (c) through a second filter with a cut-off between 50 and 300 kDa and collecting the retentate, thereby obtaining a cell lysate enriched in tailocin.

15. A cell lysate obtained by the method of claim 14.
